(19) Europäisches Patentamt / European Patent Office / Office européen des brevets

(11) **EP 3 808 371 B1**

(12) **EUROPEAN PATENT SPECIFICATION**

(45) Date of publication and mention
of the grant of the patent:
**22.11.2023 Bulletin 2023/47**

(21) Application number: **18922562.6**

(22) Date of filing: **30.10.2018**

(51) International Patent Classification (IPC):
*A61K 9/08* (2006.01)   *A61K 38/47* (2006.01)
*A61K 38/18* (2006.01)   *A61K 31/7012* (2006.01)
*A61P 27/04* (2006.01)   *A61K 9/00* (2006.01)
*A61K 31/728* (2006.01)   *A61K 47/02* (2006.01)
*A61P 27/02* (2006.01)   *A61P 31/02* (2006.01)

(52) Cooperative Patent Classification (CPC):
**A61K 38/1808; A61K 9/0048; A61K 9/08;
A61K 31/7012; A61K 31/728; A61K 38/47;
A61K 47/02; A61P 27/02; A61P 27/04;
A61P 31/02;** C12Y 302/01017

(86) International application number:
**PCT/CN2018/112517**

(87) International publication number:
**WO 2019/237633 (19.12.2019 Gazette 2019/51)**

(54) **NOVEL ARTIFICIAL TEARS CONTAINING RECOMBINANT HUMAN LYSOZYME AND RECOMBINANT HUMAN EPIDERMAL GROWTH FACTOR**

NEUARTIGE KÜNSTLICHE TRÄNEN MIT REKOMBINANTEM HUMANEM LYSOZYM UND REKOMBINANTEM MENSCHLICHEM EPIDERMALEM WACHSTUMSFAKTOR

NOUVELLES LARMES ARTIFICIELLES CONTENANT UN LYSOZYME HUMAIN RECOMBINANT ET UN FACTEUR DE CROISSANCE ÉPIDERMIQUE HUMAIN RECOMBINANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.06.2018 CN 201810613195**

(43) Date of publication of application:
**21.04.2021 Bulletin 2021/16**

(73) Proprietor: **Shaanxi Huikang Bio-Tech Co., Ltd
Xi'an City, Shaanxi Province 710077 (CN)**

(72) Inventors:
• **SHI, Jin**
  **Xi'an City, Shaanxi Province, 710077 (CN)**
• **HOU, Zengmiao**
  **Xi'an City, Shaanxi Province, 710077 (CN)**
• **GAO, En**
  **Xi'an City, Shaanxi Province, 710077 (CN)**
• **LI, Xiaoying**
  **Xi'an City, Shaanxi Province, 710077 (CN)**
• **PANG, Rongrong**
  **Xi'an City, Shaanxi Province, 710077 (CN)**
• **YANG, Xiaolin**
  **Xi'an City, Shaanxi Province, 710077 (CN)**
• **ZHAO, Jinli**
  **Xi'an City, Shaanxi Province, 710077 (CN)**

(74) Representative: **Habermann, Hruschka &
Schnabel
Patentanwälte
Montgelasstraße 2
81679 München (DE)**

(56) References cited:
**CN-A- 102 188 695      CN-A- 102 552 889
US-A1- 2007 280 924     US-A1- 2008 213 188
US-A1- 2017 246 218     US-B2- 7 655 698**

**(Cont. next page)**

- Anonymous: "Sodium hyaluronate | 9067-32-7", Chemical Book, 1 January 2017 (2017-01-01), pages 1-6, XP055753401, Retrieved from the Internet: URL:https://www.chemicalbook.com/ChemicalProductProperty_EN_CB4176691.htm [retrieved on 2020-11-24]
- SAAEHA RAUZ ET AL: "Serum eye drops, amniotic membrane and limbal epithelial stem cells-tools in the treatment of ocular surface disease", CELL AND TISSUE BANKING, KLUWER ACADEMIC PUBLISHERS, DO, vol. 11, no. 1, 22 April 2009 (2009-04-22) , pages 13-27, XP019767483, ISSN: 1573-6814

Remarks:

The complete document including Reference Table(s) and the Sequence Listing(s) can be downloaded from the EPO website

## Description

### Technical field

The present invention relates to novel artificial tears containing a recombinant human lysozyme and a recombinant human epidermal growth factor (rhEGF), belonging to the field of medicine technology.

### Background Art

Eye inflammation, dry eyes, and eye fatigue are among the most commonly seen conditions in people's daily life. With increasing work-related stress, day-by-day updatings of electronic products, and prolonged use of computers and smart phones, the chance of having dry eyes and dry eye syndrome has been increasing. According to statistics, in recent years the population of dry-eye patients living in cities has been rapidly growing at a rate of 10% to 20% per year, and the incidence is as high as 30%. Studies haven shown that people facing a computer screen for 9 hours or more every day over a long period would double the chance of having an eye disorder than those who do not; nearsighted people facing a computer for a long period is at an even higher risk of having a dry eye disorder. Normally one's eyes blink about 20 times per minute, and this number decreases to 6 if he/she is using a computer or smart phone because the eyes are very much focused and constantly adjust the focal distance to ensure clear objects to be seen. Prolonged, intensive use of eyes, in combination with various radiations from the screen of electronic devices, would have the eyes seriously irritated, resulting in reduced secretion of tears and degeneration of tear components, and in turn causing ophthalmo xerosis, together with superficial ophthalmo xerosis and uncomfortable conditions such as congestion, eye fatigue, decreased vision, and blurred vision.

Ophthalmo xerosis refers to a general term for various conditions including abnormal quality or quantity of tears or abnormal kinetics of tears caused by any reason, which results in decreased stability of the tear film, accompanied by ocular discomfort and/or characteristic pathological changes in the ocular surface tissue. Ophthalmo xerosis can be clinically graded into mild, moderate, and severe degrees, although domestically and abroad there is not yet a gold standard for its grading. However, in general, the mild degree mainly has subjective symptoms, and examinations thereof are mainly based on signs and enquires about the medical history; the moderate degree has subjective symptoms, and examinations thereof mainly involve examining the breakup time of tear film (BUT), cornea fluorescein staining (CFS), and slight keratoconjunctival epithelial injury; and the severe degree is mainly characterized by a BUT less than 5 seconds, seriously reduced tear secretion, obvious cornea and conjunctiva fluorescein staining, and damaged corneal epithelial cells.

The natural tears of human are a transparent fluid secreted by human organs like lacrimal glands, composed of many substances such as inorganic salts, polysaccharides, proteins and lipids, have barrier, bacteriostatic, bactericidal, and immunomodulatory functions, and play an important role in protecting eyeballs, nourishing the ocular surface tissue, accomplishing the visual function, etc. Current commercially available artificial tear products against tear secretion-deficient ophthalmo xerosis of various origins mainly act to supplement basic fluid and moisturize, and are characterized by simple composition and hypotonicity. Although these products can temporarily relieve eye dryness and discomfort, they also further dilute various tear components that are already at low levels on the xerophthalmic ocular surface, such as inorganic salts, lipid transfer proteins, lysozyme, lactoferrin, and the like in tears, making the tear film on the ocular surface more unstable and further weakening the bactericidal and immunomodulatory functions.

On the other hand, many commercially available artificial tear products against symptoms of eye discomfort such as eye dryness, eye irritation, and eye fatigue contain chemical bacteriostatic agents. Such products work well in short-term use, but long-term exposure to bacteriostatic agents may cause irritations such as allergy and dry eye, damage the cornea or conjunctiva and hamper their healing, and may also cause iatrogenic keratitis, etc. Numerous tests have shown that bacteriostatic agents in ophthalmic solutions have cytotoxic effects, which can affect functions and normal metabolism of cells, and long-term use thereof can damage the ocular surface tissue and lower the tolerance of eyes to these ophthalmic solutions. Some even lead to refractory drug-induced conjunctivitis and drug-induced ophthalmo xerosis. Some clinical investigations have shown that, among patients using ophthalmic solutions containing bacteriostatic agents, those having uncomfortable symptoms such as foreign body sensation, tingling sensation, and burning sensation in the eyes are 2.5 times more, and those having red eyes and dry eyes are over 2 times more, than those having the corresponding symptoms but using ophthalmic solutions free of bacteriostatic agents. After these patients switched to ophthalmic solutions free of bacteriostatic agents, these symptoms were apparently alleviated or reversed. Another investigation shows that because the irritating effect of bacteriostatic agents in ophthalmic solutions reduces the compliance of many patients, the number of eye droppings is actively reduced, thereby affecting the clinical therapeutic effects.

US20070280924A1 disclosed a pharmaceutical preparation suitable for use in the eye, which comprises: (i) a pharmaceutically acceptable carrier suitable for use in the eye; (ii) one or more ingredients selected from factors and agents that promote any one or more of survival, health, cell attachment and normal differentiation of ocular surface epithelial cells and optionally factors and agents to prevent squamous metaplasia; (iii) one or more agents capable of altering the fluid properties of a tear film including at least one agent capable of establishing and/or maintaining a stable tear film and optionally one or more agents selected from opthalmological lubricating agents, viscosity enhancing agents and agents capable of reducing tear film evaporation; the factors and agents in component (ii) and (iii) being synthetic or recombinant or licensed for pharmaceutical use.

US7655698B2 disclosed a use of L-carnitine and/or of one or more alkanoyl L-carnitines or one of their pharmaceutically acceptable salts for the preparation of an ophthalmic physiological supplement or medicament in the form of eye-drops, for the treatment of corneal diseases.

SAAEHA RAUZ ET AL (Serum eye drops, amniotic membrane and limbal epithelial stem cells-tools in the treatment of ocular surface disease, published online: 22 April 2009) disclosed that the advent of serum eye drops, amniotic membrane and limbal stem cell grafts, have transformed the treatment of diseases which result in ocular surface failure, and provided an overview of ocular surface anatomy and failure, and how the use of serum eye drops, amniotic membrane and limbal epithelial stem cell transplantation has influenced ophthalmological practice both historically and in recent years. This review focuses on the rationale for the use of these emerging tools, how they are prepared, clinical applications, limitations, and speculates on future directions.

US20080213188A1 disclosed an ophthalmic solution comprising: a) a human recombinant lysozyme; b) one or more natural lacrophyl substances; c) water; and d) optionally one or more therapeutic substances. The ophthalmic solution is useful to treat dry eye conditions and eye inflammation and also to condition and/or cleanse contact lenses.

US20170246218A1 disclosed novel ophthalmic preparations for the treatment of corneal pathologies comprising umbilical cord blood plasma.

CN102188695A disclosed an ophthalmic gel composition. Each gram of the composition comprises necessary components of: 1000 to 100000 IU (international unit) of epidermal growth factors (EGF), 0.5 to 3.5 mg of hyaluronate sodium, and rest components of pharmaceutical acceptable carriers. It also disclosed a preparation method and a purpose of the composition.

CN102552889A disclosed a biologically sterilizing eye drop preparation. The preparation comprises the following biologically sterilizing components in percentage by weight: 0.0001 to 1 percent of biologically sterilizing component, 0.001 to 10 percent of negative charge biological adhesive material and 0.001 to 10 percent of buffer solution. Compared with the traditional eye drop, the sterilizing eye drop preparation disclosed by the invention has the advantages that the bioavailability of medicaments in eyes can be obviously improved and the sterilizing efficiency of the biologically sterilizing component in the eyes can be obviously improved.

**Summary of Invention**

The invention is defined in the claims.

In order to solve the above technical problems, an objective of the present invention is to provide novel artificial tears for treatment of clinically common moderate-to-severe ophthalmo xerosis syndrome relating to visual display terminal.

To achieve the above objective, the present invention provides novel artificial tears containing a recombinant human lysozyme and an rhEGF, which comprise main components and an auxiliary material, wherein the main components are a recombinant human lysozyme, an rhEGF, and sodium hyaluronate, and the contents of the recombinant human lysozyme, the rhEGF, and the sodium hyaluronate are 0.075%-0.300%, 0.002%-0.004%, and 0.10%-0.30%, respectively, based on the total mass of the novel artificial tears; wherein the auxiliary material comprises a pH stabilizer, and the pH stabilizer is a buffer consisting of disodium hydrogen phosphate and sodium dihydrogen phosphate; the auxiliary material comprises sodium chloride, and based on the total mass of the artificial tears, the content of sodium chloride is 0.70%-0.76% as measured in terms of pure solid sodium chloride; wherein the novel artificial tears have a pH of 6.4 to 6.6 and an osmolality of 285 to 310 mOsmol/kg.

According to an embodiment of the present invention, preferably, in the novel artificial tears, the recombinant human lysozyme has an amino acid sequence completely identical to that of natural human lysozyme. Although use of lysozyme as an ocular agent has been reported, the currently used lysozyme is generally extracted from egg white, i.e., hen egg lysozyme, which is different from human lysozyme in amino acid sequence, is heterogenous to human bodies, and often causes side effects such as drug resistance, immune responses, and allergies when used on human bodies. The recombinant human lysozyme (rhLYZ) used in the present invention is obtained as white lyophilized powder by microbial fermentation, specifically by fermentation of engineered *Pichia* cells expressing the human lysozyme and purification of the expressed lysozyme. The rhLYZ has a molecular weight of 14,700 Da, purity greater than 98%, and an amino acid sequence 100% identical to that of natural human lysozyme, being homogenous to a human body. Its amino acid sequence is shown below (SEQ ID NO: 1):

```
  1   KVFERCELAR  TLKRLGMDGY  RGISLANWMC  LAKWESGYNT  RATNYNAGDR
 51   STDYGIFQIN  SRYWCNDGKT  PGAVNACHLS  CSALLQDNIA  DAVACAKRVV
101   RDPQGIRAWV  AWRNRCQNRD  VRQYVQGCGV
```

The rhEGF used according to the present invention is obtained as white lyophilized powder by microbial fermentation, specifically by fermentation of engineered *Pichia* cells expressing the human epidermal growth factor and purification of the expressed epidermal growth factor. The obtained rhEGF has purity greater than 95%, and an amino acid sequence 100% identical to that of natural human epidermal growth factor (EGF), as shown below (SEQ ID NO: 5):

```
  1   NSDSECPLSH  DGYCLHDGVC  MYIEALDKYA  CNCVVGYIGE  RCQYRDLKWW
 51   ELR
```

According to an embodiment of the invention, preferably, in the novel artificial tears, the recombinant human lysozyme is contained in an amount of 0.150% to 0.300% based on the total mass of the artificial tears.

According to an embodiment of the present invention, preferably, the novel artificial tears have a pH of 6.5.

In the novel artificial tears, the auxiliary material comprises a pH stabilizer; and the pH stabilizer is the buffer consisting of disodium hydrogen phosphate and sodium dihydrogen phosphate, so that the pH of the artificial tears can be stabilized at 6.5 to ensure an optimal antibacterial effect of the artificial tears and stability of the formulation, and meanwhile the pH is closer to the pH of natural tears and does not irritate eyes. Preferably, the disodium hydrogen phosphate is disodium hydrogen phosphate dodecahydrate, and the sodium dihydrogen phosphate is sodium dihydrogen phosphate dihydrate, and more preferably, their mass ratio is 20:9.

The novel artificial tears have an osmolality of 285 to 310 mOsmol/kg, which is isotonic with human tears. Hypertonic solutions in eyes may cause the cornea to dehydrate and aggravate the symptoms of eye dryness; hypotonic solutions may make the corneal tissue cells swell or even rupture; and isotonic solution systems can effectively preserve the normal physiological state of the eye tissue cells and alleviate the symptoms of dry eyes. The rhEGF cannot exert its function of promoting cell proliferation in a hypertonic or hypotonic environment where cells are dying, and it can only sufficiently produce its effects on viable health cells in an isotonic physiological environment.

In the novel artificial tears, the auxiliary material comprises sodium chloride. In the novel artificial tears according to the present invention, the content of sodium chloride is controlled at 0.70%-0.76% (measured in terms of pure solid sodium chloride), which can simultaneously ensure good solubility, activity and stability of the recombinant human lysozyme in the artificial tears, and an osmolality of the artificial tears of 285-310 mOsmol/kg, which is isotonic with human tears.

According to an embodiment of the present invention, preferably, based on the total amount of the novel artificial tears of 10,000-20,000 parts by weight, the novel artificial tears comprise: 7.5-60.0 parts of a recombinant human lysozyme, 0.2 to 0.8 parts of an rhEGF, 10.0-60.0 parts of sodium hyaluronate, 20.0-40.0 parts of disodium hydrogen phosphate dodecahydrate, 9.0-18.0 parts of sodium dihydrogen phosphate dihydrate, 70.0-152.0 parts of sodium chloride, and 9,840.6-19,754.6 parts of water for injection. More preferably, based on the total amount of the novel artificial tears of 10,000 parts by weight, the novel artificial tears comprise: 15.0-30.0 parts of a recombinant human lysozyme, 0.2 to 0.4 parts of an rhEGF, 10.0-30.0 parts of sodium hyaluronate, 20.0 parts of disodium hydrogen phosphate dodecahydrate, 9.0 parts of sodium dihydrogen phosphate dihydrate, 70.0-75.0 parts of sodium chloride, and 9,840.6-9,870.8 parts of water for injection.

According to an embodiment of the present invention, preferably, the novel artificial tears may have the following specific compositions:

based on the total amount of the novel artificial tears of 10,000 parts by weight, the novel artificial tears comprising: 7.5 parts of a recombinant human lysozyme, 0.2 parts of an rhEGF, 10.0 parts of sodium hyaluronate, 20.0 parts of disodium hydrogen phosphate dodecahydrate, 9.0 parts of sodium dihydrogen phosphate dihydrate, 76.0 parts of sodium chloride, and 9,877.3 parts of water for injection; or

based on the total amount of the novel artificial tears of 10,000 parts by weight, the artificial tears comprising: 15.0 parts of a recombinant human lysozyme, 0.2 parts of an rhEGF, 10.0 parts of sodium hyaluronate, 20.0 parts of disodium hydrogen phosphate dodecahydrate, 9.0 parts of sodium dihydrogen phosphate dihydrate, 75.0 parts of sodium chloride, and 9,870.8 parts of water for injection; or

based on the total amount of the novel artificial tears of 10,000 parts by weight, the artificial tears comprise: 15.0 parts of a recombinant human lysozyme, 0.2 parts of an rhEGF, 30.0 parts of sodium hyaluronate, 20.0 parts of disodium hydrogen phosphate dodecahydrate, 9.0 parts of sodium dihydrogen phosphate dihydrate, 72.0 parts of sodium chloride, and 9,853.8 parts of water for injection; or

based on the total amount of the novel artificial tears of 20,000 parts by weight, the artificial tears comprise: 60.0 parts of a recombinant human lysozyme, 0.4 parts of an rhEGF, 20.0 parts of sodium hyaluronate, 40.0 parts of disodium hydrogen phosphate dodecahydrate, 18.0 parts of sodium dihydrogen phosphate dihydrate, 148.0 parts of sodium chloride, and 19,713.6 parts of water for injection; or

based on the total amount of the novel artificial tears of 20,000 parts by weight, the artificial tears comprise: 60.0 parts of a recombinant human lysozyme, 0.8 parts of an rhEGF, 60.0 parts of sodium hyaluronate, 40.0 parts of disodium hydrogen phosphate dodecahydrate, 18.0 parts of sodium dihydrogen phosphate dihydrate, 140.0 parts of sodium chloride, and 19,681.2 parts of water for injection.

In order to address the clinically common ophthalmo xerosis syndrome relating to visual display terminal caused by reduced nictations and over-evaporation of tears due to excessive use of electronic devices such as computers and mobile phones during people's working and daily life, the present invention provides novel artificial tears having excellent effects of improving the quality and quantity of tear secretion, alleviating dry eye symptoms, and promoting repair of damaged corneal cells, mainly useful for treatment of clinically common moderate-to-severe ophthalmo xerosis syndrome relating to visual display terminal . The novel artificial tears according to the present invention are sterile, free of any chemical bacteriostatic or preservative, can be isotonic with human tears, have the function of maintaining the physiological environment at the ocular surface and suppressing bacteria, and are closer to human natural tears. The artificial tears can be packaged in daily dose units, with a single dose to be used within 24 hours, in a safe and efficacious manner.

The novel artificial tears provided by the present invention can be prepared by a method comprising the following steps:

(1) adding sodium hyaluronate to water for injection and allowing the sodium hyaluronate to be completely dissolved, to obtain a sodium hyaluronate solution;

(2) adding disodium hydrogen phosphate dodecahydrate, sodium dihydrogen phosphate dihydrate and sodium chloride to the sodium hyaluronate solution and allowing them to be completely dissolved, to obtain an auxiliary solution;

(3) adding lyophilized powder of a recombinant human lysozyme and lyophilized powder of an rhEGF to the auxiliary solution, allowing both to be completely dissolved, and then adjusting the pH of the solution to 6.5±0.1, to obtain the artificial tears;

(4) sterilizing the artificial tears by filtration.

Preferably, the method for preparation according to the present invention further comprises: (5) filling a container with the filtered and sterilized artificial tears.

The above method for preparation may be implemented in the following specific steps:

(1) preparation of sodium hyaluronate solution

adding sodium hyaluronate to water for injection in the ratio described above, and allowing the sodium hyaluronate to be completely dissolved under stirring (for example, for about 3 hours) to obtain a sodium hyaluronate solution;

(2) preparation of auxiliary solution

adding disodium hydrogen phosphate dodecahydrate, sodium dihydrogen phosphate dihydrate and sodium chloride to the sodium hyaluronate solution in the ratio described above and allowing them to be completely dissolved under stirring, to obtain an auxiliary solution;

(3) preparation of artificial tears

adding lyophilized powder of a recombinant human lysozyme and lyophilized powder of an rhEGF to the auxiliary solution in the ratio described above, allowing the lyophilized powder of the recombinant human lysozyme and the lyophilized powder of the rhEGF to be completely dissolved under stirring, and then adjusting the pH of the solution to 6.5±0.1 (for example, adjusting the pH of the solution with hydrochloric acid at a concentration of 5% (w/w)); wherein the well stirred artificial tears should have an osmolarity of 285-310 mOsmol/kg as measured with a freezing point osmometer;

(4) sterilization by filtration

sterilizing the artificial tears by sterile filtration, preferably with a 0.22 μm sterilizing filter, wherein the filtration operation pressure for the sterile filtration may be controlled at 0.35-0.40 MPa; and transferring the sterile artificial tears after the sterile filtration to a sterilized storage tank;

(5) filling

carrying out filling by a Blow-Fill-Seal (BFS) three-in-one sterile filling technique. Blow-Fill-Seal (BFS) means that in a workshop for sterile eye drop production, plastic particles are compressed and heat-melted in an injection molding machine (at 170-230°C, 350 bar (1 bar=0.1 MPa)) and then made into a plastic container, and the container is filled with a prepared sterile liquid under the protection of the A-level laminar flow by a blow-fill-seal machine and sealed, wherein the filling specification can be determined by a person skilled in the art according to actual needs, for example, the filling specification is 0.8 mL/container; wherein the blow molding of bottles, liquid filling, and formulation sealing are done in one set of continuous sterile production procedures.

In the above steps, all pipes used for material transferring must be pre-sterilized.

Most currently available artificial tears are packaged in large doses, which are repeatedly opened during use, and would easily cause secondary contamination by pathogenic microorganisms such as bacteria during long-term use. Therefore, relevant chemical bacteriostatic agents are added to ensure the quality of products during use. Extensive clinical data and research data have shown that many adverse effects seen in ophthalmic treatment are caused by bacteriostatic agents. It has been confirmed in some research that bacteriostatic agents do cause symptoms such as corneal epithelial cell damage, allergies and eye dryness. In order to ensure the safety and practicability of the artificial tears according to the present invention during use, they can be filled into packages of a daily dose, for example, 0.8 mL/bottle, which are ready to open upon use, with a single dose to be used within 24 hours. The packages of a daily dose are less likely prone to secondary contamination of the artificial tears during use.

The artificial tears according to the invention are a topical external sterile formulation, in which the recombinant human lysozyme and the rhEGF are active proteins as the main components, and the prepared raw material and auxiliary material are sterilized by a selected terminal filtration means, and the sterile liquid is used to fill a container under sterile conditions. This ensures the eye drops be a sterile formulation and also ensures the activity of the recombinant human lysozyme and the rhEGF, effectively avoiding inactivation of the recombinant human lysozyme and the rhEGF easily caused by high temperature sterilization.

As compared with the prior art, the technical solution of the present invention has the following beneficial effects: the artificial tears according to the present invention comprise a recombinant human lysozyme, an rhEGF, and sodium hyaluronate as the main components, do not contain any chemical bacteriostatic agent, do not cause any damage to eyes or allergic reactions during long-term use, and ate safe and efficacious.

The present invention effectively combines a recombinant human lysozyme, an rhEGF, sodium hyaluronate and an auxiliary material together, mainly for treatment of moderate-to-severe ophthalmo xerosis, and upon external administration to eyes, it shows significant therapeutic and ameliorating effects on decreased tear secretion, eye dryness, slight inflammation, cornea injury and the like caused by moderate-to-severe ophthalmo xerosis.

**Description of Drawings**

Figures 1a shows the result of fluorescein sodium staining of corneal epithelium of rats in the sodium hyaluronate control group before establishing a model.

Figures 1b shows the result of fluorescein sodium staining of corneal epithelium of rats in the recombinant human lysozyme control group before establishing a model.

Figures 1c shows the result of fluorescein sodium staining of corneal epithelium of rats in the rhEGF control group before establishing a model.

Figures 1d shows the result of fluorescein sodium staining of corneal epithelium of rats in the artificial tear group before establishing a model.

Figures 2a shows the result of fluorescein sodium staining of corneal epithelium of rats in the sodium hyaluronate control group 10 days after establishing a model.

Figures 2b shows the result of fluorescein sodium staining of corneal epithelium of rats in the recombinant human lysozyme control group 10 days after establishing a model.

Figures 2c shows the result of fluorescein sodium staining of corneal epithelium of rats in the rhEGF control group 10 days after establishing a model.

Figures 2d shows the result of fluorescein sodium staining of corneal epithelium of rats in the artificial tear group 10 days after establishing a model.

Figures 3a shows the result of fluorescein sodium staining of corneal epithelium of rats in the sodium hyaluronate control group 5 days after treatment.

Figures 3b shows the result of fluorescein sodium staining of corneal epithelium of rats in the recombinant human lysozyme control group 5 days after treatment.

Figures 3c shows the result of fluorescein sodium staining of corneal epithelium of rats in the rhEGF control group 5 days after treatment.

Figures 3d shows the result of fluorescein sodium staining of corneal epithelium of rats in the artificial tear group 5 days after treatment.

Figure 4a shows the result (400×) of HE staining of pathological slides of corneal epithelium of rats in the sodium hyaluronate control group 5 days after treatment.

Figure 4b shows the result (400×) of HE staining of pathological slides of corneal epithelium of rats in the recombinant human lysozyme control group 5 days after treatment.

Figure 4c shows the result (400×) of HE staining of pathological slides of corneal epithelium of rats in the rhEGF control group 5 days after treatment.

Figure 4d shows the result (400×) of HE staining of pathological slides of corneal epithelium of rats in the artificial tear group 5 days after treatment.

## Detailed Description of Invention

In order to provide a better understanding of the technical features, objectives and advantageous effects of the present invention, the technical solutions of the present invention will be described below in details, but the detailed description below should not be construed as limiting the implementable scope of the present invention.

The artificial tears according to the present invention were obtained by the inventors through intensive theoretical and experimental research with creative efforts. Although the artificial tears according to the present invention are not completely identical to natural human tears, they have the basic characteristics of tears, including maintaining the physiological environment at the ocular surface (moistening and moisturizing eyeballs, being isotonic and stable), having a bacteriostatic function and a function of promoting proliferation of corneal epithelial cells (the test in Example 13 shows that the artificial tears have both a bacteriostatic function and a cell proliferation-promoting function), and having a natural origin (being sterile and free of chemical bacteriostatic agent), etc., and can exerts the basic functions of natural tears. In the composition of the artificial tears according to the present invention, the selection and ratio of the components lead to a good synergistic effect to promote each other.

Firstly, the recombinant human lysozyme, as one of the main components of the artificial tears, is a mucopolysaccharide lyase, which is a non-specific immune factor present in normal body fluid and tissues of a human, and also an important component of the human eye immune defense system, having an antibacterial and anti-inflammatory effect. External dropwise addition of human lysozyme can better protect eyes from bacterial infection and inflammation. Moreover, human lysozyme is an intrinsic substance in natural tears of a human body, and long-term use thereof would not cause any toxic side effects to the human body.

Another effective active ingredient in the novel artificial tears according to the present invention is an rhEGF. Human EGF is an important growth factor secreted by human body. It is widely found in various human tissues and can promote the division and growth of epidermal cells. EGF has a function of promoting growth of cells such as keratinocytes, fibroblasts, smooth muscle cells, and endothelial cells, and is also an important cytokine for repair of damaged corneal epithelium and stroma. It can effectively repair corneal damage.

Sodium hyaluronate is a non-Newtonian fluid having good biocompatibility, which increases the viscosity of the medicine while overcoming the disadvantage of difficulty in blinking of eyelids. Moreover, sodium hyaluronate has a good moisturizing and lubricating effect, and can make patients with ophthalmo xerosis feel moisturized, refreshed and comfortable in eyes. Under normal circumstances, hyaluronic acid naturally present on the ocular surface forms a film covering the surface of the corneal epithelium, and the corneal epithelium has specific binding sites for hyaluronic acid.

The above three main components are optimally combined in the present invention. The sodium hyaluronate contained in the artificial tears forms a film covering the surface of the corneal epithelium, so that the recombinant human lysozyme and the rhEGF can maintain at the ocular surface for a long time, where the recombinant human lysozyme exerts a bacteriostatic function and reduces invasion of exogenous pathogenic microorganisms into the corneal epithelial cells, and the rhEGF promotes repair and regeneration of damaged corneal epithelial cells. The test of pharmacodynamic effects of the artificial tears on a benzalkonium chloride-induced rat dry-eye model accompanied by severe corneal epithelial damage given in Example 16 demonstrates that the artificial tears according to the present invention have therapeutic effects significantly better than the control containing sodium hyaluronate as the only active component, the control containing recombinant human lysozyme as the only active component, and the commercially available eye drops product containing EGF as the only active component.

Secondly, when a plurality of active proteins or main components are combined together, the main substances may interact with each other and result in decreased efficacy of each substance, in which case the therapeutic effect of the combination is even less than that of each of the individual components. The three main components in the present invention can exert an excellent combinational effect, in which the choice and composition of auxiliaries also play an important role in addition to the characteristics of the three main components *per se*.

During the preparation, purification and test of the recombinant human lysozyme, the inventors have surprisingly found that the activity and stability of the recombinant human lysozyme are in a proportional relationship with the sodium chloride content in the solution. The higher the content of the recombinant human lysozyme in the solution, the higher the proportion of sodium chloride required for complete dissolution. The experimental study on the solubility of recombinant human lysozyme and the content of sodium chloride in a solution in Example 5 confirms that, when the content by weight of recombinant human lysozyme is 0.300%, the dissolution stability of the recombinant human lysozyme decreases and turns into a suspension state, with both content and activity decreased, as the salt concentration decreases in a sodium chloride solution at a concentration of 0.6% or lower, but the dissolution stability and activity of the recombinant human lysozyme maintains substantially constant in a sodium chloride solution at a concentration from 0.6% to 6%. In the artificial tears according to the present invention, the content of sodium chloride by weight may be controlled at 0.70%-0.76%, which can simultaneously ensure good solubility, activity and stability of the recombinant human lysozyme in the artificial tears, and also an osmolality of the artificial tears of 285-310 mOsmol/kg, which is isotonic with human tears.

pH is a very important technical indicator in artificial tears, which is related to the stability, effectiveness and irritation to eyes of ophthalmic formulations. The recombinant human lysozyme as an active ingredient has an isoelectric point of 9.24, and shows bacteriostatic activity at pH 5.0-8.0. The experimental study on the bacteriostatic activity and pH of the recombinant human lysozyme given in Example 6 shows that the pH corresponding to the best bacteriostatic activity is 6.5; and the active component rhEGF has an isoelectric point of 4.67, and can sufficiently exert its cell proliferation-promoting function on viable health cells in a physiological environment. The ratio of disodium hydrogen phosphate-sodium dihydrogen phosphate used in the present invention can stabilize the pH of eye drops at $6.5\pm0.1$, which is substantially close to the physiological pH of human and ensures the conditions for optimal activity of the recombinant human lysozyme and the rhEGF, and this pH is different from the isoelectric points of the recombinant human lysozyme and the rhEGF by 1.5 pH units or more, which ensures long-term stability of the recombinant human lysozyme and the rhEGF, without causing any irritation to human eyes.

An auxiliary solution is formed with the above choice and ratios of the three inorganic salts, and is combined with the three main components to obtain the artificial tears, which are demonstrated to be a stable formulation in Example 13 (the test of combinational stability of recombinant human lysozyme and rhEGF in artificial tears), Example 14 (Stress test under enforced high-frequency vibration of artificial tears), and Example 15 (Long-term stability and accelerated test of artificial tears). These components function synergistically, and the test of pharmacodynamic effects of the artificial tears on a benzalkonium chloride-induced rat dry-eye model accompanied by severe corneal epithelial damage given in Example 16 demonstrates that the artificial tears are definitely efficacious and show better effects of improving the quantity of tear secretion and promoting repair of damaged corneal cells than the controls each containing a single active component.

Example 1. Preparation of recombinant human lysozyme

1. Construction of strains for recombinant human lysozyme

1.1 Construction of expression vector

According to the sequence of human lysozyme (LYZ) published in GenBank, primers were designed with the Primer Premier 5.0 software (the primer sequences are shown in Table 1), wherein the restriction endonuclease Xho I restriction site CTCGAG and the Kex2 restriction site AAAAGA were introduced in the forward primer, and the restriction endonuclease EcoR I site GAATTC was introduced in the reverse primer. The nucleotide sequences after introduction of the aforementioned sites are as follows (SEQ ID NO: 2):

```
  1 CTCGAGAAAA GAAAGGTCTT TGAAAGGTGT GAGTTGGCCA GAACTCTGAA AAGATTGGGA
 61 ATGGATGGCT ACAGGGGAAT CAGCCTAGCA AACTGGATGT GTTTGGCCAA ATGGGAGAGT
121 GGTTACAACA CACGAGCTAC AAACTACAAT GCTGGAGACA GAAGCACTGA TTATGGGATA
181 TTTCAGATCA ATAGCCGCTA CTGGTGTAAT GATGGCAAAA CCCCAGGAGC AGTTAATGCC
241 TGTCATTTAT CCTGCAGTGC TTTGCTGCAA GATAACATCG CTGATGCTGT AGCTTGTGCA
301 AAGAGGGTTG TCCGTGATCC ACAAGGCATT AGAGCATGGG TGGCATGGAG AAATCGTTGT
361 CAAAACAGAG ATGTCCGTCA GTATGTTCAA GGTTGTGGAG TGTAAGAATT C
```

Table 1. Primer sequences and annealing temperature

| Primer | Primer sequences (5'→3') | Extension length/bp | Annealing temperature /°C |
|---|---|---|---|
| LYZ-XLT | GCCTCGAGAAAAGAAAGGTCTTTGAAAGGTGTGA ( SEQ ID NO:3) | 415 | 56 |
| LYZ-EL | CGGAATTCTTACACTCCACAACCTTGAA ( SEQ ID NO:4) | | |

The human skin cDNA was used as a template, and the designed LYZ-XU and LYZ-EL were used as primers to perform PCR with an annealing temperature of 56°C. The PCR results showed specific bands at expected positions, and the target bands were recovered with a DNA purification and recovery kit. The recovered DNA products, together with the pPIC9K vector (purchased from Invitrogen), were double digested with Xho I (purchased from Thermo Fisher) and EcoR I (purchased from Thermo Fisher), and the digested products were recovered and ligated with DNA ligase, and transformed into *Escherichia coli*. The plasmid was extracted and identified by sequencing, to obtain a human lysozyme expression vector, which was named pPIC9K-LYZ.

1.2 Electrotransformation of *Pichiapastoris*

10 µg pPIC9K-LYZ plasmid linearized with Sal I endonuclease was mixed with 80 µL of competent *Pichia pastoris* cells, transferred to a 0.2 cm electrotransformation cell pre-cooled on ice, electrically shocked for 4-10 ms, and added to a 1 mL solution of 1 mol/L sorbitol pre-cooled on ice. The cells were thoroughly mixed and plated onto an MD (13.4 g/L amino-free yeast nitrogen source (YNB), 20 g/L glucose (Dextrose), and 0.4 mg/L biotin) medium plate, which was inverted and cultured at 30°C for 3-4 days. Colonies grew on the MD medium plate.

1.3 Screening for recombinants with multiple copies of insertions

The colonies grown on the MD medium plate were inoculated with sterilized toothpicks to YPD (1wt% Yeast Extract, 2wt% polypeptone, and 2wt% Dextrose) plates with a G418 concentration of 1 g/L, 2 g/L, 3 g/L, and 4 g/L, respectively, incubated at 30°C, and screened through shaking flasks to obtain the transformants.

2. Fermentation culture and induced expression of recombinant human lysozyme 2.1 Primary seed culturing

The transformants obtained from screening were inoculated separately into 4 flasks each containing 250 mL of a BMGY medium (1% yeast extract; 2% peptone; 100 mmol/L potassium phosphate solution, pH 6.0; 1.34% YNB (Yeast nitrogen base); 1% glycerol), incubated in a thermostatic shaker at 29°C, 225 rpm for 60-70 h, and then inoculated to a seed tank.

2.2 Secondary seed culturing

The primary seeds were transferred to a seed tank containing 3 L of an FBS medium (containing a mixture of 40 g glycerin, 18.2 g $K_2SO_4$, 26.7 mL $H_3PO_4$, 0.93 g $CaSO_4 \cdot 2H_2O$, 14.9 g $MgSO_4$, and 4.13 g KOH per liter), and cultured at a tank temperature controlled at 29.0±1.0°C, a tank pressure controlled at 0.050±0.010 MPa, and a pH controlled at 5.0. The aeration and stirring speed during the culturing were adjusted to maintain the dissolved oxygen at about 30%. After 16 hours of secondary seed culturing, dissolved oxygen was observed and found to increase significantly (10% increase within 1.0 min). The secondary seed culturing was completed. The seed culturing period is generally 16-24 hours.

2.3 Fermentation in a 150 L fermenter

After the secondary seed culturing was completed, the seeds were transferred to a 150 L fermenter containing 90 L FBS medium, and were cultured at a culture temperature controlled at 29.0±1.0°C, a fermenter pressure controlled at 0.050±0.010 MPa, a DO of about 30% controlled by manually adjusting the aeration volume, oxygen volume and rotation speed. Upon culturing for 15-20 h, the substrates were depleted, and the dissolved oxygen rose sharply, indicating start of the feeding stage. Immediately after the start of the feeding stage, oxygen supply was turned off and the stirring speed was lowered to reduce the DO to about 40%. The automatic feeding system was started, and the initial flow rate of a glycerin solution was 1.7 mL/min (1 s/60 s). The fed-batch rate of the glycerin feed was adjusted according to the actual fermentation state, and a GPE antifoam agent was manually supplemented according to the actually foaming state. After feeding for 20 h, samples were taken to measure the wet cell weight of the fermentation broth. When the wet cell weight of the fermentation broth reached 260 g/L, the glycerin feed was stopped and starvation was started.

After the glycerin feed was stopped, the supply of carbon source was insufficient, and the dissolved oxygen again rose significantly. The aeration volume was adjusted and the rotation speed was lowered to control the DO at 30%-40%, to initiate the starvation stage which was controlled to last for 1.0 h. After the starvation was completed, the automatic feeding system was started, the initial feed flow rate was 1.7 mL/min (1 s/60 s), after 3 hours the flow rate was increased to 3.4 mL/min (2 s/60 s), after 6 hours the flow rate was increased to 5.1 mL/min (3 s/60 s), and after 9 hours the methanol induction stage was started. The fed-batch rate of methanol was increased according to the actual level of DO. The fed-batch rate of methanol was generally controlled at up to 13.6 mL/min (8 s/60 s), and the methanol induction period was generally controlled at 40-48 h. In the methanol induction phase, the DO should be controlled at 20%-35% and it should be ensured that the methanol accumulation be not excess. When the induction time reached 40-48 h or the methanol supplement volume reached 20-30 L, the methanol induction phase was completed and the product was removed from the fermenter.

3 Purification of recombinant human lysozyme

3.1 Crude purification of recombinant human lysozyme

The fermentation broth was centrifuged in a decanter centrifuge at 2000 rpm for 90 minutes for solid-liquid separation; the supernatant was collected, in which solid sodium chloride was added and completely dissolved to give a final concentration of 0.8 M-1.0 M, and then passed through a 0.2 μm hollow fiber membrane filtration system to remove residual yeasts and cell debris, especially the green pigments in the fermentation broth of *Pichia pastoris*. The 0.2 μm filtrate was collected and subjected to phenyl hydrophobic chromatography for fine purification.

3.2 Fine purification of recombinant human lysozyme

3.2.1 Purification by hydrophobic chromatography: Preparation of mobile phase A: 1 M sodium chloride solution; phase B: purified water plus 10% isopropanol, at a pH adjusted to 8.0 with a 0.5 M sodium hydroxide solution. Phase A was used to equilibrate a Phenyl Sepharose 6 FF (GE company, USA) high-flow phenyl hydrophobic sepharose gel chromatography column. When equilibrium was reached and the conductivity was 80 mS/cm, the collected 0.2 μm filtrate was loaded onto the column which was then washed with flowing phase A for about 3 column bed volumes, and when the ultraviolet absorption was reduced to below 500 mV, the column was eluted with phase B and the eluate was collected.

3.2.2 Sample processing: The eluate collected in 3.2.1 was adjusted to pH 7.6-7.8 with a 1 M sodium dihydrogen phosphate solution, and the conductivity was 6.0-7.0 mS/cm.

3.2.3 Purification by CM cation exchange chromatography: preparation of mobile phase A: 20 mM phosphate buffer, pH 7.6-7.8, conductivity 2.0-2.3 mS/cm; mobile phase B: 20 mM phosphate buffer, 1 M NaCl, pH 7.6-7.8. The recombinant human lysozyme sample in 3.2.2 was loaded onto a CM Sepharose FF (GE company, USA) high-flow cation exchange sepharose gel chromatography column. After all the sample was loaded, phase A was used to equilibrate the column for 2-3 column bed volumes, a gradient to 16% phase B was applied to remove impurities, then the gradient was adjusted to 25% phase B to carry out elution, and the eluate was collected as the recombinant human lysozyme.

The eluate was purified by Sephadex G25 (GE Company, USA) gel chromatography, and the solution containing the protein peak was collected and freeze-dried to obtain lyophilized powder of the recombinant human lysozyme with a purity of 98% or more.

Example 2: Measurement of quality indices of recombinant human lysozyme

1. Characteristics

The recombinant human lysozyme prepared in Example 1 was white or off-white amorphous powder; odorless; easily destroyed by alkali; easily dissolved in a 0.6% or higher sodium chloride aqueous solution; and insoluble in acetone or diethyl ether.

2. Identification

(1) about 2 mg of the recombinant human lysozyme prepared in Example 1 was dissolved in 2 drops of added physiological saline, and 5 drops of a 10% sodium hydroxide solution and 1 drop of a 10% copper sulfate solution were added and well mixed, resulting in a magenta appearance.

(2) about 2 mg of the recombinant human lysozyme prepared in Example 1 was added to an acetic acid-sodium acetate buffer (6.7 g of anhydrous sodium acetate was added to about 900 mL water, and dissolved under shaking; the pH was adjusted to 5.4 with acetic acid; the solution was diluted to 1000 mL with water, followed by thorough shaking) to make a solution containing 0.2 mg of lysozyme per 1 mL. According to the spectrophotometric method (Pharmacopoeia of People's Republic of China, the 2015 edition, Part III, General rule 0401), the maximum absorption at 280 nm was measured as 0.42 to 0.52.

(3) In the chromatogram recorded as an assay item in the purity analysis, the retention time of the main peak of the test sample solution was consistent with the retention time of the main peak of the control solution.

(4) According to the immunoblotting method (Pharmacopoeia of People's Republic of China, the 2015 edition, Part III, General rule 3401), it was positive.

3. Molecular weight

According to the mass spectrometry method (Pharmacopoeia of People's Republic of China, the 2015 edition, Part IV, General rule 0431), by MALDI-TOF, the molecular weight of the recombinant human lysozyme was measured as 14700 D $\pm$ 100 D. The molecular weight of the recombinant human lysozyme prepared in Example 1 was 14700 D.

4. Isoelectric point

According to the capillary isoelectric focusing electrophoresis method (Pharmacopoeia of People's Republic of China, the 2015 edition, Part III, General rule 0542: capillary electrophoresis method), the isoelectric point of the recombinant human lysozyme was 9.24.

5. Peptide map

According to the peptide mapping method (Pharmacopoeia of People's Republic of China, the 2015 edition, Part III, General rule 3405: the first method: trypsin lysis-reversed phase high performance liquid chromatography), the peptide map of the sample was consistent with that of the control.

6. N-terminal amino acid sequence

The sequence of the 15 amino acids at the N-terminus of the recombinant human lysozyme was: Lys-Val-Phe-Glu-Arg-Cys-Glu-Leu-Ala-Arg-Thr-Leu-Lys-Arg-Leu.

7. Residual methanol

According to the gas chromatography (Pharmacopoeia of People's Republic of China, the 2015 edition, Part III, General rule 0521: gas chromatography), the methanol content was not higher than 0.002%.

8. Residual exogenous DNA

According to the residual exogenous DNA assay method (Pharmacopoeia of People's Republic of China, the 2015 edition, Part III, General rule 3407), residual exogenous DNA per 100 $\mu$g protein should not exceed 10 ng.

9. Residual host protein

According to the assay method for residual protein from engineered yeast cells (Pharmacopoeia of People's Republic of China, the 2015 edition, Part III, General rule 3414), the residual host protein was not higher than 0.1% of total protein.

10. pH

Physiological saline was added to 0.1 g of the recombinant human lysozyme prepared in Example 1 to make 10 mL to dissolve the lysozyme, and the pH was 6.5-8.5 as measured by the pH measuring method (Pharmacopoeia of People's Republic of China, the 2015 edition, Part III, General rule 0631).

11. Weight loss on drying

0.2 g of the recombinant human lysozyme prepared in Example 1 was assayed according to the moisture measuring method (Pharmacopoeia of People's Republic of China, the 2015 edition, Part III, General rule 0832), and the weight loss on drying was not more than 10.0%.

12. Heavy metal (Pb)

According to the second method for measuring heavy metal (Pharmacopoeia of People's Republic of China, the 2015 edition, Part III, General rule 0821), the heavy metal was not more than 10 parts per million.

13. Residue on ignition

0.2 g of the recombinant human lysozyme prepared in Example 1 was assayed according to the method for measuring residue on ignition (Pharmacopoeia of People's Republic of China, the 2015 edition, Part III, General rule 0841), and the residue was not more than 4.0%.

14. Total protein content

The recombinant human lysozyme prepared in Example 1 was assayed according to the protein content measuring method (Pharmacopoeia of People's Republic of China, the 2015 edition, Part III, General rule 0731: the second method: Foline-phenol method), and the total nitrogen content was not less than 90% based on dry samples.

15. Purity

(1) According to the non-reducing SDS-polyacrylamide gel electrophoresis method (Pharmacopoeia of People's Republic of China, the 2015 edition, Part III, General rule 0541: the fifth method), the content of the recombinant human lysozyme was $\geq$ 95.0%.
(2) According to the reversed-phase high performance liquid chromatography-the area normalization method (Pharmacopoeia of People's Republic of China, the 2015 edition, Part III, General rule 0512), the area of the main peak of the recombinant human lysozyme was not smaller than 95% of the total area according to area normalization, wherein the sample loading amount was not less than 5 $\mu$g, the detection was carried out at a wavelength of 280 nm, and the theoretical plate number was not less than 16000 as calculated based on the chromatographic peaks of the recombinant human lysozyme.

16. Biological Activity - Titer measurement

(1) Reagent preparation: phosphate buffer (0.02 M/L, pH 6.5): 3.115 g of $Na_2HPO_4 \cdot 12H_2O$, 1.763 g of $NaH_2PO_4 \cdot 2H_2O$, and 6.0 g of NaCl were weighed out and added to 800 mL of distilled water to be dissolved, and then made up to 1000 mL.

Substrate suspension: 20 mg *of Micrococcus lysodeikticus* powder was weighed out, added to 0.5 mL of phosphate buffer, and soaked for 1 hour. Then an appropriate amount of phosphate buffer was added such that the absorbance of the suspension measured at 450 nm at 25°C was 0.800 ± 0.05 (prepared right before use).

Recombinant human lysozyme solution: 10 mg of recombinant human lysozyme was precisely weighed out, placed in a 5 mL volumetric flask, made up to 5 mL with a phosphate buffer, and diluted to obtain a solution of recombinant human lysozyme at 200 $\mu$g/mL.

(2) Operation

0.1 mL of the recombinant human lysozyme solution at a concentration of 200 $\mu$g/mL was pipetted and added at room temperature 25 °C and pH 6.5 into a cuvette containing 3 mL of substrate suspension. The cuvette was placed in an ultraviolet spectrophotometer, the absorbance was measured at 450 nm, and the titer was calculated. A decrease in absorbance of 0.001 per minute represents one unit of enzyme activity. A sample group and a control group were set up. 3 mL of substrate suspension was precisely measured out at 25±0.1°C, and placed in a 1 cm cuvette. The absorbance was measured at 450 nm, and the readings at zero second were recorded as $A_0$ and $A'_0$. Then 0.1 mL of phosphate buffer and 0.1 mL of the 200 $\mu$g/mL recombinant human lysozyme solution were precisely measured out, added to the cuvette, and quickly mixed, and the time was counted with a stopwatch. At 60 seconds, the absorbance values $A_{60}$ and $A'_{60}$ were read, and the activity titer was calculated with the following equation:

$$\textbf{Activity titer}\ (\text{U/mg})\ = \frac{(A_0 - A_{60}) - (A'_0 - A'_{60})}{W} \times 10^6$$

wherein W ($\mu$g) is the weight of the test sample in the measurement liquid.

The measurement was repeated 3 times, and the average value thereof was used to calculate the unit activity of the recombinant human lysozyme which should be not less than $1.0 \times 10^5$ U/mg.

17. Microbial limit

Total number of bacteria (CFU/g): 0.5 g was taken for measurement according to the microbiological limit measuring method for non-sterile products (Pharmacopoeia of People's Republic of China, the 2015 edition, Part III, General rule 1105). The total number of bacteria (CFU/g) <100.

The total number of mould and yeasts (CFU/g): 0.5 g was taken for measurement according to the microbiological limit measuring method for non-sterile products (Pharmacopoeia of People's Republic of China, the 2015 edition, Part III, General rule 1105). The total number of mould and yeasts (CFU/g) <10.

18. Endotoxin

According to the endotoxin measuring method (Pharmacopoeia of People's Republic of China, the 2015 edition, Part III, General rule 1143), endotoxin per 1 mg lyophilized powder of recombinant human lysozyme was less than 10 EU.

After the examinations, the recombinant human lysozyme prepared in Example 1 met the standards and was a qualified product, and can be further used for preparing artificial tears.

Example 3: Preparation of recombinant human EGF (rhEGF)

1. Construction of strains for rhEGF

1.1 Construction of expression vector

According to the amino acid sequence of hEGF published in GenBank, primers were artificially designed based on the coden preference in *Pichia pastoris* and synthesized (Shanghai Sangon Biotech), wherein the restriction endonuclease Xho I restriction site CTCGAG and the Kex2 restriction site AAAAGA were introduced at the 5' end, and the restriction endonuclease Not I site GCGGCCGC was introduced at the 3' end. The nucleotide sequences after introduction of the aforementioned sites are as follows (SEQ ID NO: 6):

```
  1   CTCGAGAAAA GAAACTCTGA CTCTGAATGT CCATTGTCTC ACGACGGTTA CTGTTTGCAC
 61   GACGGTGTTT GTATGTACAT CGAAGCTTTG GACAAGTACG CTTGTAACTG TGTTGTTGGT
121   TACATCGGTG AAAGATGTCA ATACAGAGAC TTGAAGTGGT GGGAATTGAG ATAAGCGGCC
181   GC
```

The hEGF gene synthesized from the whole genome sequence and the pPIC9K vector (purchased from Invitrogen) were double digested with Xho I (purchased from Thermo Fisher) and Not I (purchased from Thermo Fisher), and the digested products were recovered and ligated with DNA ligase, and transformed into *Escherichia coli*. The plasmid was extracted and identified by sequencing, to obtain an hEGF expression vector, which was named pPIC9K-EGF.

1.2 Electrotransformation of *Pichia pastoris*

10 μg pPIC9K-EGF plasmid linearized with Sal I endonuclease was mixed with 80 μL of competent *Pichia pastoris* cells, transferred to a 0.2 cm electrotransformation cell pre-cooled on ice, electrically shocked for 4-10 ms, and added to a 1 mL solution of 1 mol/L sorbitol pre-cooled on ice. The cells were thoroughly mixed and plated onto an MD medium (13.4 g/L amino-free yeast nitrogen source (YNB), 20 g/L glucose (Dextrose), and 0.4 mg/L biotin) plate, which was inverted and cultured at 30°C for 3-4 days. Colonies grew on the MD medium plate.

1.3 Screening for recombinants with multiple copies of insertions

The colonies grown on the MD medium plate were inoculated with sterilized toothpicks to YPD (1% Yeast Extract, 2% polypeptone, and 2% Dextrose) plates with a G418 concentration of 1 g/L, 2 g/L, 3 g/L, and 4 g/L, respectively, incubated at 30°C, and screened through shaking flasks to obtain the transformants.

2. Fermentation culture and induced expression of rhEGF

2.1 Primary seeds culturing

The transformants obtained from screening were inoculated separately into 4 flasks each containing 250 mL of a BMGY medium (1% yeast extract; 2% peptone; 100 mmol/L potassium phosphate solution, pH 6.0; 1.34% YNB (Yeast nitrogen base); 1% glycerol), incubated in a thermostatic shaker at 29°C and 225 rpm for 60-70 h, and then inoculated to a seed tank.

2.2 Secondary seeds culturing

The primary seeds were transferred to a seed tank containing 3 L of an FBS medium (containing a mixture of 40 g glycerin, 18.2 g $K_2SO_4$, 26.7 mL $H_3PO_4$, 0.93 g $CaSO_4 \cdot 2H_2O$, 14.9 g $MgSO_4$, and 4.13 g KOH per liter), and cultured at a tank temperature controlled at $29.0 \pm 1.0°C$, a tank pressure controlled at $0.050 \pm 0.010$ MPa, and a pH controlled at 5.5. The aeration and stirring speed during the culturing were adjusted to maintain the dissolved oxygen at about 30%. After 16 hours of secondary seeds culturing, dissolved oxygen was observed and found to increase significantly (10% increase in 1.0 min). The secondary seeds culturing was completed. The seeds culturing period is generally 16-24 hours.

2.3 Fermentation in a 150 L fermenter

After the secondary seeds culturing was completed, the seeds were transferred to a 150 L fermenter containing 90 L FBS medium, and were cultured at a culture temperature controlled at $29.0 \pm 1.0°C$, a fermenter pressure controlled at $0.050 \pm 0.010$ MPa, a DO of about 30% controlled by manually adjusting the aeration volume, oxygen volume and rotation speed, and a pH controlled at 5.5. Upon culturing for 15-20 h, the substrates were depleted, and the dissolved oxygen rose sharply, indicating start of the feeding stage. Immediately after the start of the feeding stage, oxygen supply was turned off and the stirring speed was lowered to reduce the DO to about 40%. The automatic feeding system was started, and the initial flow rate of a glycerin solution was 1.7 mL/min (1 s/60 s). The fed-batch rate of the glycerin feed was adjusted according to the actual fermentation state, and a GPE antifoam agent was manually supplemented according to the actually foaming state. After feeding for 20 h, samples were taken to measure the wet cell weight of the fermentation broth. When the wet cell weight of the fermentation broth reached 300 g/L, the glycerin feed was stopped and starvation was started.

After the glycerin feed was stopped, the supply of carbon source was insufficient, and the dissolved oxygen again rose significantly. The aeration volume was adjusted and the rotation speed was lowered to control the DO at 30%-40%, to initiate the starvation stage which was controlled to last for 1.0 h. After the starvation was completed, the automatic feeding system was started, the initial feed flow rate was 1.7 mL/min (1 s/60 s), after 3 hours the flow rate was increased to 3.4 mL/min (2 s/60 s), after 6 hours the flow rate was increased to 5.1 mL/min (3 s/60 s), and after 9 hours the methanol induction stage was started. The fed-batch rate of methanol was increased according to the actual level of DO. The fed-batch rate of methanol was generally controlled at up to 13.6 mL/min (8 s/60 s), and the methanol induction period was generally controlled at 40-48 h. In the methanol induction phase, the DO should be controlled at 20-35% and it should be ensured that the methanol accumulation be not excess. When the induction time reached 45-52 h or the methanol supplement volume reached 25-30 L, the methanol induction phase was completed and the product was removed from the fermenter.

3 Purification of rhEGF

3.1 Crude purification of rhEGF

The fermentation broth was centrifuged in a decanter centrifuge at 2000 rpm for 90 minutes for solid-liquid separation; the supernatant was collected and centrifuged, and then passed through a 0.2 $\mu$m hollow fiber membrane filtration system to remove residual yeasts and cell debris. The 0.2 $\mu$m filtrate was collected, and subjected to de-salting and concentrating in a 1 KD rolled ultrafilter film-based system. The ultrafiltration was completed when the concentrated volume reached about 3 L and the conductivity reached 0.6$\pm$0.1 mS/cm, and the concentrate was collected for fine purification.

3.2 Fine purification of rhEGF

3.2.1 Sample processing I: The concentrate collected in 3.1 was adjusted to a pH of 5.4-5.6 with a 1 M sodium dihydrogen phosphate solution and a 0.5 M sodium hydroxide solution, where the conductivity was 2.0 mS/cm or lower.
3.2.2 Purification I by CM cation exchange chromatography: preparation of mobile phase A: 20 mM phosphate buffer, pH 5.4-5.6, conductivity 1.3-1.5 mS/cm; mobile phase B: 20 mM phosphate buffer, 1 M NaCl, pH 5.4-5.6. A CM Sepharose FF (GE company, USA) high-flow cation exchange sepharose gel chromatography column was equilibrated with phase A for 3-5 column bed volumes. After the equilibration was complete, the sample obtained in 3.2.1 was loaded onto the column, and the flow-through was collected as an rhEGF fraction. Afterwards the column was washed with the flow of phase A for about 3 column bed volumes until the UV absorbance reached 200 mV or lower, and was eluted with 100% phase B. The eluate contained protein impurities.
3.2.3 Sample processing II: The flow-through collected in 3.2.2 was adjusted to a pH of 4.2-4.3 with a 1 M citric acid solution, and the conductivity was adjusted to 1.5 mS/cm or lower with a small amount of purified water.
3.2.4 Purification II by CM cation exchange chromatography: preparation of mobile phase A: 20 mM sodium citrate buffer, pH 4.2-4.3, conductivity 1.3-1.5 mS/cm; mobile phase B: 20 mM sodium citrate buffer, 1 M NaCl, pH 4.2-4.3. A CM Sepharose FF (GE company, USA) high-flow cation exchange sepharose gel chromatography column was equilibrated with phase A for 3-5 column bed volumes. After the equilibration was complete, the rhEGF sample obtained in 3.2.3 was loaded onto the column, and afterwards the column was equilibrated with phase A for 2-3 column bed volumes, eluted with a gradient to 5% phase B to remove impurities, and further eluted with a gradient to 16% phase B. The eluate was collected as an rhEGF fraction.
3.2.5 The eluate obtained in 3.2.4 was adjusted to a pH of 6.3-6.5 with a 0.5 M sodium hydroxide solution, and purified by Sephadex G25 (GE company, USA) gel chromatography. The solution containing the protein peak was collected and freeze-dried to obtain lyophilized powder of the rhEGF with a purity of 95% or more.

Example 4: Measurement of quality indices of rhEGF

1. Characteristics

The rhEGF prepared in Example 3 was white or off-white amorphous powder, odorless. When in an aqueous solution, it is easily destroyed by alkali.

2. Identification

(1) about 2 mg of the rhEGF prepared in Example 3 was dissolved in 2 drops of added water, and then 5 drops of a 10% sodium hydroxide solution and 1 drop of a 10% copper sulfate solution were added and well mixed, resulting in a magenta appearance.

(2) about 10 mg of the rhEGF prepared in Example 3 was diluted to 1.0 mg/ml with water, and scanned within the wavelength range from 240 to 320 nm according to the spectrophotometric method (Pharmacopoeia of People's Republic of China, the 2015 edition, Part III, General rule 0401). The maximum absorption peak should appear at 276 nm

(3) According to the immunoblotting method (Pharmacopoeia of People's Republic of China, the 2015 edition, Part III, General rule 3401), it should be positive.

3. Molecular weight

According to the mass spectrometry method (Pharmacopoeia of People's Republic of China, the 2015 edition, Part IV, General rule 0431), by MALDI-TOF, the molecular weight of the rhEGF was measured as 6200 D $\pm$ 50 D. The molecular weight of the rhEGF prepared in Example 3 was 6200 D.

4. Isoelectric point

According to the capillary isoelectric focusing electrophoresis method (Pharmacopoeia of People's Republic of China, the 2015 edition, Part III, General rule 0542: capillary electrophoresis method), the isoelectric point of the rhEGF was 4.67.

5. Peptide map

According to the peptide mapping method (Pharmacopoeia of People's Republic of China, the 2015 edition, Part III, General rule 3405: the first method: trypsin lysis-reversed phase high performance liquid chromatography), the peptide map of the sample should be consistent with that of the reference.

6. N-terminal amino acid sequence

The sequence of the 15 amino acids at the N-terminus of the rhEGF was: Asn-Ser-Asp-Ser-Glu-Cys-Pro-Leu-Ser-His-Asp-Gly-Tyr-Cys-Leu.

7. Residual methanol

According to the gas chromatography (Pharmacopoeia of People's Republic of China, the 2015 edition, Part III, General rule 0521: gas chromatography), the methanol content should not be higher than 0.002%.

8. Residual exogenous DNA

According to the residual exogenous DNA assay method (Pharmacopoeia of People's Republic of China, the 2015 edition, Part III, General rule 3407), residual exogenous DNA per 100 $\mu$g protein should not exceed 10 ng.

9. Residual host protein

According to the assay method for residual protein from engineered yeast cells (Pharmacopoeia of People's Republic of China, the 2015 edition, Part III, General rule 3414), the residual host protein should not be higher than 0.1% of total protein.

10. pH

Water was added to 0.1 g of the rhEGF prepared in Example 3 to make 10 mL to dissolve the rhEGF, and the pH should be 5.0-7.0 as measured by the pH measuring method (Pharmacopoeia of People's Republic of China, the 2015 edition, Part III, General rule 0631).

11. Weight loss on drying

0.2 g of the rhEGF prepared in Example 3 was assayed according to the moisture measuring method (Pharmacopoeia of People's Republic of China, the 2015 edition, Part III, General rule 0832), and the weight loss on drying should not exceed 10.0%.

## 12. Heavy metal (Pb)

According to the second method for measuring heavy metal (Pharmacopoeia of People's Republic of China, the 2015 edition, Part III, General rule 0821), the heavy metal should not be more than 10 parts per million.

## 13. Residue on ignition

0.2 g of the rhEGF prepared in Example 3 was assayed according to the method for measuring residue on ignition (Pharmacopoeia of People's Republic of China, the 2015 edition, Part III, General rule 0841), and the residue should not exceed 4.0%.

## 14. Total protein content

The rhEGF prepared in Example 3 was assayed according to the protein content measuring method (Pharmacopoeia of People's Republic of China, the 2015 edition, Part III, General rule 0731: the second method: Foline-phenol method), and the total nitrogen content should not be less than 90%.

## 15. Purity

(1) According to the non-reducing SDS-polyacrylamide gel electrophoresis method (Pharmacopoeia of People's Republic of China, the 2015 edition, Part III, General rule 0541: the fifth method), the content of the rhEGF should be $\geq$ 95.0%.
(2) According to the reversed-phase high performance liquid chromatography-the area normalization method (Pharmacopoeia of People's Republic of China, the 2015 edition, Part III, General rule 0512), the area of the main peak of the rhEGF should not be smaller than 95% of the total area according to area normalization, wherein the sample loading amount was not less than 5 $\mu$g, the detection was carried out at a wavelength of 280 nm, and the theoretical plate number was not less than 1000 as calculated based on the chromatographic peaks of the rhEGF.

## 16. Biological Activity - Cell proliferation method/MTT colorimetry

According to the assay method for biological activity of rhEGF (Pharmacopoeia of People's Republic of China, the 2015 edition, Part III, General rule 3528), the biological activity of the rhEGF was assayed based on the fact that rhEGF stimulates growth of mice embryonic fibroblasts (BALB/c 3T3 cells) and the growth status of BALB/c 3T3 cells vary depending on the biological activity of rhEGF.

## (1) Reagent preparation

PRMI 1640 medium: One bag of PRMI 1640 medium powder (specification: 1 L) was dissolved in water and diluted to 1000 ml, to which $10^5$ IU penicillin and $10^5$ IU streptomycin and then 2.1 g sodium bicarbonate were added, dissolved, and well mixed. The mixture was sterilized by filtration, and stored at 4°C.

Maintenance medium: The RPMI 1640 medium was added to 4 mL fetal bovine serum (FBS) to make 1000 mL.

Complete medium: The RPMI 1640 medium was added to 100 mL FBS to make 1000 mL.

PBS: 8 g of sodium chloride, 0.2 g of potassium chloride, 1.44 g of disodium hydrogen phosphate, and 0.24 g of potassium dihydrogen phosphate were dissolved in water and diluted to 1000 ml, and sterilized at 121°C for 15 minutes.

Thiazolyl Blue Tetrazolium (MTT) solution: 0.10 g MTT powder was dissolved in 20 ml PBS, sterilized by filtration through a 0.22 $\mu$m filter, and stored at 4°C in darkness.

Preparation of standard solutions: A standard of rhEGF was reconstituted according to the instructions, and diluted with the maintenance medium to 50 IU/mL. In a 96-well cell culture plate, 4-fold serial dilutions were performed to make 8 dilutions in total, with 2 wells for each concentration. The above operations were carried out under sterile conditions.

Preparation of test samples: The test sample was reconstituted according to the indicated amount, and diluted with the maintenance medium to about 50 IU/mL. In a 96-well cell culture plate, 4-fold serial dilutions were performed to make 8 dilutions in total, with 2 wells for each concentration. The above operations were carried out under sterile conditions.

(2) Assay method

A BALB/c 3T3 cell line was cultured in the complete medium at 37°C and 5% $CO_2$, and the cell density was controlled at $1.0\times10^5$ to $5.0\times10^5$ cells/ml. 24-36 hours after passage, the cells were assayed for biological activity. The medium in the culture flask was discarded. The cells were digested, collected, and suspended in the complete medium at $5.0\times10^4$ to $8.0\times10^4$ cells/ml. The cell suspension was inoculated in a 96-well cell culture plate with 100 $\mu$l/well, and cultured at 37°C and 5% $CO_2$. After 24 hours, the medium was replaced with the maintenance medium, and cultured at 37°C and 5% $CO_2$ for 24 hours. The maintenance medium was discarded from the resultant cell culture plate, and a standard solution and a test sample solution were added with 100 $\mu$l per well, and incubated at 37°C and 5% $CO_2$ for 64-72 hours. 20 $\mu$l of the MTT solution was added to each well, and incubated at 37°C and 5% $CO_2$ for 5 hours. The above operations were carried out under sterile conditions. After the liquid in the culture plate was discarded, 100 $\mu$L of dimethyl sulfoxide was added to each well, and well mixed. Then the absorbance was read at the wavelength of 570 nm on a microplate reader with a reference wavelength of 630 nm, and the assay results were recorded.

The test data was processed by computer program or four-parameter regression calculation, and the result was calculated by the following equation:

$$\text{Biological activity (IU/ml) of the test sample} = Pr \times Ds \times Es / Dr / Er$$

wherein Pr is the biological activity of the standard in IU/mL;
Ds is the pre-dilution factor of the test sample;
Dr is the pre-dilution factor of the standard;
Es is the dilution factor of the test sample equivalent to 50% effect dose of the standard;
Er is the dilution factor of the standard having 50% effect dose.

The assay was repeated 3 times, and the average value thereof was used to calculate the ratio of the biological activity to the protein content, which should be not less than $5.0\times10^5$ IU/mg protein.

17. Microbial limit

Total number of bacteria (CFU/g): 0.5 g was taken for measurement according to the microbiological limit measuring method for non-sterile products (Pharmacopoeia of People's Republic of China, the 2015 edition, Part III, General rule 1105). The total number of bacteria (CFU/g) <100.

The total number of fungi and yeasts (CFU/g): 0.5 g was taken for measurement according to the microbiological limit measuring method for non-sterile products (Pharmacopoeia of People's Republic of China, the 2015 edition, Part III, General rule 1105). The total number of fungi and yeasts (CFU/g) <10.

18. Endotoxin

According to the endotoxin measuring method (Pharmacopoeia of People's Republic of China, the 2015 edition, Part III, General rule 1143), endotoxin per 1 mg lyophilized powder of rhEGF should be less than 10 EU.

After the examinations, the rhEGF prepared in Example 3 met the standards and was a qualified product, and can be further used for preparing artificial tears.

Example 5: Study on the solubility of recombinant human lysozyme and the content of sodium chloride in solution

1. Materials and apparatus

1.1 Test materials: the lyophilized powder of recombinant human lysozyme prepared according to

Example 1

1.2 Test apparatus: Ultraviolet spectrophotometer, and an electronic balance with a precision of 0.0001 g.

2. Test method

9 batches of 0.0300 g lyophilized powder of recombinant human lysozyme were precisely weighed out on the electronic balance, and placed in nine 10-mL volumetric flasks, respectively. To the volumetric flasks, water for injection and solutions of sodium chloride at a mass concentration of 0.2%, 0.4%, 0.6 %, 0.8%, 1.0%, 2.0%, 4.0%, and 6.0% were respectively added to the volume mark, to obtain 9 solutions of recombinant human lysozyme at a concentration of 3.0 mg/mL theoretically, which were allowed to stand for 1 hour for full dissolution. The state of the solutions was observed. An appropriate amount of each of the 9 solutions was filtered with a 0.22 $\mu$m filter, an appropriate amount of each of the filtrates was taken for measurement of the protein content and the bacteriostatic activity of the 9 samples, according to the total protein amount measuring method and the biological activity-titer measuring method described in Example 2.

3. Test results

The test results for the solubility of recombinant human lysozyme and the sodium chloride content in the solution are summarized in Table 2. It can be seen from the test data that the solutions having a sodium chloride content below 0.6% (0.4%, 0.2%, and water for injection) were a non-clear transparent liquid, generally in a state of suspension; as the salt concentration decreased, the suspension state of the recombinant human lysozyme increased gradually, with the most apparent suspension state of the recombinant human lysozyme found in pure water. At the same time, the protein content and bacteriostatic activity also showed a decreasing trend, mainly because the recombinant human lysozyme was not fully dissolved in solutions having a sodium chloride content below 0.6%. The lower the sodium chloride content, the lower the solubility. The undissolved proteins were filtered off by passing through a 0.22 $\mu$m filter during measurement, and thus only the dissolved proteins in the filtrate and their corresponding bacteriostatic activity were measured. The results also show that the recombinant human lysozyme may be dissolved in pure water in a small amount, and the solubility is limited. In solutions containing 0.6% or more of sodium chloride, 3.0 mg/mL of recombinant human lysozyme were completely dissolved, and the protein content and bacteriostatic activity were stable and consistent.

Table 2. Summary of the results for the solubility of recombinant human lysozyme and the sodium chloride content in the solution

| | Sodium chloride content in solutions of recombinant human lysozyme (%) | | | | | | | | |
|---|---|---|---|---|---|---|---|---|---|
| | 0 | 0.2 | 0.4 | 0.6 | 0.8 | 1.0 | 2.0 | 4.0 | 6.0 |
| State of solution | Suspension | Suspension | Slight suspension | Clear and transparent | Clear and transparent | Clear and transparent | Clear and transparent | Clear and transparent | Clear and transparent |
| Protein content mg/mL | 0.38 | 1.21 | 2.28 | 3.06 | 3.05 | 3.07 | 3.04 | 3.04 | 3.07 |
| Bacteriostatic activity U/mL | $0.53 \times 10^5$ | $1.59 \times 10^5$ | $3.06 \times 10^5$ | $4.30 \times 10^5$ | $4.28 \times 10^5$ | $4.32 \times 10^5$ | $4.33 \times 10^5$ | $4.30 \times 10^5$ | $4.26 \times 10^5$ |

Example 6: Dependency of the bacteriostatic activity of recombinant human lysozyme on pH

1. Materials and apparatus

1.1 Test materials: the lyophilized powder of recombinant human lysozyme prepared according to

Example 1

1.2 Test apparatus: Ultraviolet spectrophotometer, and an electronic balance with a precision of 0.0001 g.

2. Test method

7 batches of 0.0300 g lyophilized powder of recombinant human lysozyme were precisely weighed out on the electronic balance, and placed in seven 10-mL volumetric flasks, respectively. To the volumetric flasks, phosphate buffers at a pH of 5.0, 5.5, 6.0, 6.5, 7.0, 7.5, and 8.0 (solutions of 0.02 M phosphate and 0.10 M sodium chloride) were respectively added to the volume mark, to obtain 7 solutions of recombinant human lysozyme at a concentration of 3.0 mg/mL but different pHs, which were each diluted into solutions of recombinant human lysozyme at 1.5 mg/ml and 0.75 mg/mL with a phosphate buffer at a corresponding pH, so as to obtain solutions of recombinant human lysozyme at three different concentrations, with each concentration corresponding to 7 different pHs. The bacteriostatic activity of the 21 samples was measured according to the biological activity-titer measuring method described in Example 2 (in the measuring method, the phosphate buffers were prepared with disodium hydrogen phosphate and sodium dihydrogen phosphate weighed according to the various pHs in this test).

3. Test results

The results of the bacteriostatic activity of recombinant human lysozyme under different pH conditions are summarized in Table 3. It can be seen from the test data that at the same pH the recombinant human lysozyme contents of 0.75 mg/mL, 1.5 mg/mL, and 3.0 mg/mL proportionally correlated with the bacteriostatic activity; the same content displayed activity at different pHs, with the best bacteriostatic activity of recombinant human lysozyme found at pH 6.5 and less bacteriostatic activity found at pH 6.0. When the pH was higher or lower than 6.5, the antibacterial activity showed a decreasing trend as the pH changed. As the pH increased and approached the isoelectric point of recombinant human lysozyme 9.24, the solubility and stability decreased, and the bacteriostatic activity decreased more significantly.

Table 3. Summary of the results of the bacteriostatic activity of recombinant human lysozyme and pH

| Concentration /pH | Bacteriostatic activity (U/mL) | | | | | | |
|---|---|---|---|---|---|---|---|
| | pH5.0 | pH5.5 | pH6.0 | pH6.5 | pH7.0 | pH7.5 | pH8.0 |
| 3.0mg/mL | $3.68\times10^5$ | $3.89\times10^5$ | $4.24\times10^5$ | $4.40\times10^5$ | $4.12\times10^5$ | $4.03\times10^5$ | $3.11\times10^5$ |
| 1.5mg/mL | $1.70\times10^5$ | $1.87\times10^5$ | $2.04\times10^5$ | $2.15\times10^5$ | $2.00\times10^5$ | $1.89\times10^5$ | $1.48\times10^5$ |
| 0.75mg/mL | $0.79\times10^5$ | $0.85\times10^5$ | $0.98\times10^5$ | $1.04\times10^5$ | $0.93\times10^5$ | $0.87\times10^5$ | $0.67\times10^5$ |

The sources of raw materials used in the following Examples 7 to 11 are as follows:

The recombinant human lysozyme was the recombinant human lysozyme prepared in Example 1;
The rhEGF was the rhEGF prepared in Example 3;
Sodium hyaluronate was purchased from Bloomage Freda Biopharm Co., Ltd., as the eye drop-grade crude sodium hyaluronate, Chinese medicine approval No. H20113379;
Disodium hydrogen phosphate dodecahydrate, sodium dihydrogen phosphate dihydrate, and sodium chloride were purchased from Sinopharm;
Water for injection was made by the applicant by a conventional method.

Example 7

The artificial tears of this Example had a composition shown in Table 4.
The artificial tears of this Example were prepared by the following method:

(1) weighing out 10.0 g sodium hyaluronate and adding it to 9870.8 g water for injection, stirring for 3 hours to allowing the sodium hyaluronate to be completely dissolved, to obtain a sodium hyaluronate solution for further use;

(2) separately weighing out 20.0 g disodium hydrogen phosphate dodecahydrate, 9.0 g sodium dihydrogen phosphate dehydrate, and 75.0 g sodium chloride, adding them to the sodium hyaluronate solution, and allowing these auxiliary materials to be completely dissolved under stirring, to obtain an auxiliary solution for further use;

(3) weighing out 15.0 g lyophilized powder of the recombinant human lysozyme and 0.2 g lyophilized powder of the rhEGF, and adding them to the auxiliary solution, followed by stirring until the lyophilized powder was completely dissolved, and then adjusting the pH of the solution to $6.5 \pm 0.1$ with 5% (w/w) hydrochloric acid; wherein the well stirred artificial tears should have an osmolarity of 285-310 mOsmol/kg as measured with a freezing point osmometer;

(4) sterilizing the artificial tears by sterile filtration with a 0.22 $\mu$m sterilizing filter at a filtration operation pressure of 0.35-0.40 MPa; and then transferring the sterile medicine liquid to a sterilized storage tank;

(5) in a workshop for sterile eye drop production, compressing and heat-melting plastic particles in an injection molding machine (at 170-230°C, 350 bar (1 Bar=0.1 MPa)) and making a plastic container therefrom, and filling the container with the sterile medicine liquid prepared in step (4) under the protection of the A-level laminar flow by a blow-fill-seal machine and sealing the container, wherein the filling specification is 0.8 mL/container; then examining, packaging and storing the filled sterile product.

The artificial tears of this Example were in packages of a daily dose, which are ready to open before use, with a single dose to be used within 24 hours.

Examples 8-11

The artificial tears of Examples 8-11 had compositions shown in Table 4.

The artificial tears of Examples 8-11 were prepared by the same method as in Example 7, expect that the amounts of components were accordingly adjusted according to the compositions shown in Table 4.

Table 4 (unit: g)

| | Example 7 | Example 8 | Example 9 | Example 10 | Example 11 |
|---|---|---|---|---|---|
| Recombinant human lysozyme | 15.0 | 60.0 | 7.5 | 15.0 | 60.0 |
| rhEGF | 0.2 | 0.8 | 0.2 | 0.2 | 0.4 |
| Sodium hyaluronate | 10.0 | 60.0 | 10.0 | 30.0 | 20.0 |
| $Na_2HPO_4 \cdot 12H_2O$ | 20.0 | 40.0 | 20.0 | 20.0 | 40.0 |
| $NaH_2PO_4 \cdot 2H_2O$ | 9.0 | 18.0 | 9.0 | 9.0 | 18.0 |
| Sodium chloride | 75.0 | 140.0 | 76.0 | 72.0 | 148.0 |
| Water for injection | 9870.8 | 19681.2 | 9877.3 | 9853.8 | 19713.6 |

Example 12: Measurement of quality indices of artificial tears

1. Appearance
The artificial tears prepared in Examples 7-11 were all colorless clear liquid.

2. pH
A 10 ml sample from each Example was assayed according to the pH measuring method (Pharmacopoeia of People's Republic of China, the 2015 edition, Part III, General rule 0631), and the pH was $6.5 \pm 0.1$.

3. Osmolarity
A 1 ml sample from each Example was assayed in a freezing point osmometer according to the osmolarity measuring method (Pharmacopoeia of People's Republic of China, the 2015 edition, Part III, General rule 0632), and the osmolarity was 285-310 mOsmol/kg.

4. Visible foreign bodies
According to the method for examining visible foreign bodies (the first method: lamp test) (Pharmacopoeia of People's Republic of China, the 2015 edition, Part III, General rule 0904), the product met the regulations for ophthalmic liquid formulations.

5. Load

The labelled load was 0.8 mL/container, the average load was not less than the labelled load, and the load in each container was not less than the labelled load. According to the method (Pharmacopoeia of People's Republic of China, the 2015 edition, Part III, General rule 0105), 10 test samples were taken and their contents were poured into a standard volumetric cylinder, followed by visual examination. Each load was not less than the labelled load.

6. The biological activity of the recombinant human lysozyme was measured according to the method described in Example 2, and upon examination, the biological activity of the recombinant human lysozyme in the artificial tears was 70%-200% of the labeled value.

7. The biological activity of the rhEGF was measured according to the method described in Example 4, and upon examination, the biological activity of the rhEGF in the artificial tears was always 70%-200% of the labeled value.

8. Measurement of the sodium hyaluronate content

(1) Preparation of control solution: 60 mg of a glucuronic acid control dried to a constant weight at 60°C and reduced pressure was precisely weighed out, placed in a 100 mL volumetric flask, dissolved in water and diluted to the mark, followed by thorough shaking; 10 mL was precisely measured out, placed in a 100 mL volumetric flask, diluted to the mark with water, and shaken well.

(2) Preparation of the sample solution: from 1-2 artificial tear containers, 0.7 ml was precisely measured out and placed in a 10 mL volumetric flask, diluted to the mark with water for injection, and shaken well.

(3) Preparation of a standard curve: 0, 0.2, 0.4, 0.6, 0.8, and 1.0 mL control solutions were precisely measured out, each placed in a 25 mL test tube equipped with a stopple, made up to 1.0 ml with water, mixed thoroughly, and cooled in an ice bath. Under continuous shaking, 5.0 mL of 0.025 mol/L borax sulfuric acid solution was slowly added dropwise, and the tube was stoppled, heated in a boiling water bath for 10 minutes (shaking it once during the heating), and cooled rapidly. A 0.2 ml solution of 0.125% carbazole in anhydrous ethanol was added, shaken well, heated in a boiling water bath for 15 minutes (shaking it once during the heating), and cooled. According to the ultraviolet-visible spectrophotometric method (Pharmacopoeia of People's Republic of China, the 2015 edition, Part III, General rule 0401), with the 0 ml tube as a blank, the absorbance was measured at 530 nm, and the regression equation was calculated for the corresponding absorbance with the number of $\mu$g of glucuronic acid.

(4) Assay: 1 mL of the sample solution was precisely measured out, placed it in a 25 mL test tube equipped with a stopple, and assayed according to the method described in "Preparation of a standard curve" from the "cooled in an ice bath" step. The content of glucuronic acid was calculated by the regression equation, and multiplied by 2.0675 to obtain the content.

The measurement was repeated 3 times, and the average value thereof was used to calculate the content of sodium hyaluronate. The sodium hyaluronate content should be between 90% and 110% of the labeled amount.

9. Sterility examination

After processing by membrane filtration, sterility was examined by the sterility examination method (Pharmacopoeia of People's Republic of China, the 2015 edition, Part III, General rule 1101), and it should be sterile.

After the examinations, the artificial tears of Examples 7 to 11 were all qualified products.

Example 13: Experimental study on stability of the combination of recombinant human lysozyme and rhEGF in artificial tears

1. Materials

Test sample 1: a blank basic liquid prepared according to Example 7, but free of recombinant human lysozyme and rhEGF;

Test sample 2: prepared according to Example 7 with addition of recombinant human lysozyme alone, free of rhEGF;

Test sample 3: prepared according to Example 7 with addition of rhEGF alone, free of recombinant human lysozyme;

Test sample 4: prepared according to Example 7.

2. Test method

According to the quality standards for artificial tears, test samples 1-4 were assayed for the appearance, pH, osmolarity, sterility, bacteriostatic activity, and cell proliferation promoting activity. The results are summarized in Table 5.

Table 5. Test results of bacteriostatic activity and proliferation-promoting activity of test samples

| Sample | Appearance | pH | Osmolarity (mOsmol/kg) | Sterility | Bacteriostatic activity (U/mL) | Cell proliferation promoting activity (ICT/mL) |
|---|---|---|---|---|---|---|
| Test sample 1 | Colorless clear liquid | 6.52 | 297.6 | sterile | - | - |
| Test sample 2 | Colorless clear liquid | 6.50 | 300.0 | sterile | $2.12 \times 10^5$ | - |
| Test sample 3 | Colorless clear liquid | 6.50 | 297.6 | sterile | - | 14000 |
| Test sample 4 | Colorless clear liquid | 6.52 | 298.4 | sterile | $2.15 \times 10^5$ | 14000 |

3. Results

The results show that upon the examination the appearance, pH, osmolarity, and sterility of test samples 1-4 were qualified and had no difference; test sample 1 had no bacteriostatic activity or cell proliferation promoting activity; test sample 2 was not significantly different from test sample 4 in terms of bacteriostatic activity; test sample 3 was not significantly from test sample 4 in terms of cell proliferation promoting activity. That is, the combination of the two biological active proteins, i.e. the recombinant human lysozyme and the rhEGF, has excellent stability in the composite formulation prepared under the conditions according to the present invention.

Example 14. Stress test under high-frequency vibration of artificial tears

In a destructive stress test, the stability of the two active proteins in the artificial tears under high-frequency vibration was investigated. An artificial tear sample prepared according to Example 7 was placed in a constant temperature shaker at 25°C and continuously shaken at 200 rpm, and was regularly sampled every week to examine the appearance, pH, osmolarity, sterility, bacteriostatic activity, and cell proliferation promoting activity. The results are summarized in Table 6.

Table 6. Results of stability test of artificial tears during vibration

| Time | Temp. | Appearance | pH | Osmolarity (mOsmol/kg) | Sterility | Bacteriostatic activity (U/mL) | Cell proliferation promoting activity (IU/mL) |
|---|---|---|---|---|---|---|---|
| Week 0 | - | Colorless clear liquid | 6.52 | 298.4 | sterile | $2.15 \times 10^5$ | 14000 |
| Week 1 | 25°C | Colorless clear liquid | 6.50 | 300.0 | sterile | $2.15 \times 10^5$ | 14000 |
| Week 2 | 25°C | Colorless clear liquid | 6.50 | 298.4 | sterile | $2.17 \times 10^5$ | 15000 |

(continued)

| Time | Temp. | Appearance | pH | Osmolarity (mOsmol/kg) | Sterility | Bacteriostatic activity (U/mL) | Cell proliferation promoting activity (IU/mL) |
|---|---|---|---|---|---|---|---|
| Week 3 | 25°C | Colorless clear liquid | 6.52 | 298.4 | sterile | $2.16 \times 10^5$ | 15000 |
| Week 4 | 25°C | Colorless clear liquid | 6.50 | 300.0 | sterile | $2.16 \times 10^5$ | 14000 |
| Week 5 | 25°C | Colorless clear liquid | 6.48 | 299.2 | sterile | $2.16 \times 10^5$ | 12000 |
| Week 6 | 25°C | Colorless clear liquid | 6.48 | 299.2 | sterile | $1.94 \times 10^5$ | 12000 |
| Week 7 | 25°C | Colorless clear liquid | 6.48 | 300.0 | sterile | $1.77 \times 10^5$ | 7000 |
| Week 8 | 25°C | Colorless clear liquid | 6.48 | 299.2 | sterile | $1.23 \times 10^5$ | 6000 |

After a 8-week destructive stress test under high-frequency vibration, the bacteriostatic activity of the recombinant human lysozyme in the artificial tears started to decline from week 6, the cell proliferation promoting activity of the rhEGF started to decline from week 5, and the other indicators showed no obvious changes. Therefore, long-term high-frequency vibration should be avoided during transportation of the artificial tears. As an overall evaluation result, the combination of the recombinant human lysozyme and the rhEGF in the present artificial tears has excellent stability within 4 weeks under high-frequency vibration.

Example 15: Long-term stability and accelerated test of artificial tears

The artificial tears were tested for stability under the storage conditions prescribed for commercialization, and the stability characteristics of the artificial tears during storage were investigated to provide a basis for determining the period of validity and storage conditions. The artificial tear sample prepared in Example 7 was separately stored at 4°C, 25°C, and 37° C in darkness at humidity of 45%-65%. Samples were taken regularly for examination of the appearance, pH, osmolarity, sterility, bacteriostatic activity, and cell proliferation promoting activity. Samples for the long-term stability test were tested once a month for the first 6 months and then once every 3 months for the next 6 months in the first year, and tested once every 6 months in the second year. Samples for the 37°C accelerated test were tested every month. The results of the stability tests at 4°C, 25°C and 37°C are summarized in Table 7, Table 8 and Table 9, respectively.

Table 7. Summary of the results of long-term stability test of artificial tears (4°C)

| Time | Temp. | Appearance | pH | Osmolarity (mOsmol/kg) | Sterility | Bacteriostatic activity (U/mL) | Cell proliferation promoting activity (IU/mL) |
|---|---|---|---|---|---|---|---|
| 0 month | - | Colorless clear liquid | 6.52 | 298.4 | Sterile | $2.15 \times 10^5$ | 14000 |
| 1 month | 4°C | Colorless clear liquid | 6.48 | 300.0 | Sterile | $2.15 \times 10^5$ | 14000 |
| 2 months | 4°C | Colorless clear liquid | 6.50 | 299.2 | Sterile | $2.17 \times 10^5$ | 15000 |
| 3 months | 4°C | Colorless clear liquid | 6.48 | 298.4 | Sterile | $2.16 \times 10^5$ | 14000 |
| 4 months | 4°C | Colorless clear liquid | 6.50 | 300.0 | Sterile | $2.16 \times 10^5$ | 15000 |
| 5 months | 4°C | Colorless clear liquid | 6.48 | 300.0 | Sterile | $2.15 \times 10^5$ | 14000 |

(continued)

| Time | Temp. | Appearance | pH | Osmolarity (mOsmol/kg) | Sterility | Bacteriostatic activity (U/mL) | Cell proliferation promoting activity (IU/mL) |
|---|---|---|---|---|---|---|---|
| 6 months | 4°C | Colorless clear liquid | 6.48 | 299.2 | Sterile | $2.12 \times 10^5$ | 14000 |
| 9 months | 4°C | Colorless clear liquid | 6.50 | 298.4 | Sterile | $2.16 \times 10^5$ | 13000 |
| 12 months | 4°C | Colorless clear liquid | 6.52 | 298.4 | Sterile | $2.15 \times 10^5$ | 14000 |
| 18 months | 4°C | Colorless clear liquid | 6.48 | 297.6 | Sterile | $2.15 \times 10^5$ | 14000 |
| 24 months | 4°C | Colorless clear liquid | 6.48 | 299.2 | Sterile | $2.16 \times 10^5$ | 14000 |

Table 8. Summary of the results of long-term stability test of artificial tears (25°C)

| Time | Temp. | Appearance | pH | Osmolarity (mOsmol/kg) | Sterility | Bacteriostatic activity (U/mL) | Cell proliferation promoting activity (IU/mL) |
|---|---|---|---|---|---|---|---|
| 0 month | - | Colorless clear liquid | 6.52 | 298.4 | Sterile | $2.15 \times 10^5$ | 14000 |
| 1 month | 25°C | Colorless clear liquid | 6.48 | 299.2 | Sterile | $2.16 \times 10^5$ | 14000 |
| 2 months | 25°C | Colorless clear liquid | 6.52 | 299.2 | Sterile | $2.16 \times 10^5$ | 15000 |
| 3 months | 25°C | Colorless clear liquid | 6.50 | 301.6 | Sterile | $2.16 \times 10^5$ | 14000 |
| 4 months | 25°C | Colorless clear liquid | 6.50 | 300.0 | Sterile | $2.15 \times 10^5$ | 15000 |
| 5 months | 25°C | Colorless clear liquid | 6.50 | 300.0 | Sterile | $2.17 \times 10^5$ | 14000 |
| 6 months | 25°C | Colorless clear liquid | 6.48 | 299.2 | Sterile | $2.13 \times 10^5$ | 14000 |
| 9 months | 25°C | Colorless clear liquid | 6.50 | 298.4 | Sterile | $2.15 \times 10^5$ | 14000 |
| 12 months | 25°C | Colorless clear liquid | 6.48 | 299.2 | Sterile | $2.16 \times 10^5$ | 14000 |
| 18 months | 25°C | Colorless clear liquid | 6.50 | 297.6 | Sterile | $2.16 \times 10^5$ | 14000 |
| 24 months | 25°C | Colorless clear liquid | 6.50 | 300.0 | Sterile | $2.15 \times 10^5$ | 13000 |

Table 9. Summary of the results of accelerated stability test of artificial tears (37°C)

| Time | Temp. | Appearance | pH | Osmolarity (mOsmol/kg) | Sterility | Bacteriostatic activity (U/mL) | Cell proliferation promoting activity (IU/mL) |
|---|---|---|---|---|---|---|---|
| 0 month | - | Colorless clear liquid | 6.52 | 298.4 | Sterile | $2.15 \times 10^5$ | 14000 |
| 1 month | 37°C | Colorless clear liquid | 6.50 | 298.4 | Sterile | $2.13 \times 10^5$ | 14000 |
| 2 months | 37°C | Colorless clear liquid | 6.52 | 298.4 | Sterile | $2.15 \times 10^5$ | 15000 |
| 3 months | 37°C | Colorless clear liquid | 6.52 | 298.4 | Sterile | $2.16 \times 10^5$ | 14000 |
| 4 months | 37°C | Colorless clear liquid | 6.50 | 301.6 | Sterile | $2.16 \times 10^5$ | 15000 |
| 5 months | 37°C | Colorless clear liquid | 6.48 | 299.2 | Sterile | $2.15 \times 10^5$ | 14000 |
| 6 months | 37°C | Colorless clear liquid | 6.48 | 299.2 | Sterile | $2.15 \times 10^5$ | 13000 |
| 7 months | 37°C | Colorless clear liquid | 6.52 | 298.4 | Sterile | $2.13 \times 10^5$ | 12000 |
| 8 months | 37°C | Colorless clear liquid | 6.50 | 303.2 | Sterile | $2.10 \times 10^s$ | 8000 |
| 9 months | 37°C | Colorless clear liquid | 6.50 | 316.4 | Sterile | $1.83 \times 10^5$ | 5000 |
| 10 months | 37°C | Colorless clear liquid | 6.48 | 322.4 | Sterile | $1.52 \times 10^5$ | 2000 |
| 11 months | 37°C | Colorless clear liquid | 6.46 | 336.6 | Sterile | $1.05 \times 10^5$ | - |
| 12 months | 37°C | Colorless clear liquid | 6.46 | 355.2 | Sterile | $1.02 \times 10^5$ | - |

RESULTS: The results of the 24-month long-term stability test show that the sample placed at 4°C for 24 months exhibited no significant difference in various parameters as compared to the 0 month sample; the sample placed at 25°C for 24 months exhibited slightly decreased, but still acceptable, cell proliferation promoting activity of the rhEGF as compared to the 0 month sample. The results of the accelerated test up to 12 months show that the samples placed at 37°C exhibited decreased bacteriostatic activity of the recombinant human lysozyme and increased osmolarity from the 8th month on, as compared to the 0 month sample; and the cell proliferation promoting activity of the rhEGF of the samples started to decrease from the 6th month on, and generally became not qualified at the 9th month. Therefore, the present artificial tears had good stability at 25°C or lower during 24-month storage, and under such conditions, the production, transportation and long-term storage will not adversely affect the quality of the product, and can ensure the safety and efficacy for clinical medication.

Example 16: Test of pharmacodynamic effects of the artificial tears on a benzalkonium chloride-induced rat dry-eye model accompanied by severe corneal damage

1. Materials and reagents

1.1 Test materials

1.1.1 The sodium hyaluronate control solution (prepared according to Example 7, without addition of recombinant human lysozyme or rhEGF)

Name: sodium hyaluronate control solution;
Specification: 0.8 mL/bottle;
Recombinant human lysozyme: 0 mg;
rhEGF: 0 mg;
Sodium hyaluronate: 0.8 mg;
Appearance: colorless transparent clear liquid
Storage condition: stored at room temperature (25°C or lower).

1.1.2 The recombinant human lysozyme control solution (prepared according to Example 7, without addition of hyaluronate or rhEGF)

Name: recombinant human lysozyme control solution;
Specification: 0.8 mL/bottle;
Recombinant human lysozyme: 1.2 mg: 120000U;
rhEGF: 0 mg/ml;
Sodium hyaluronate: 0 mg;
Appearance: colorless transparent clear liquid
Storage condition: stored at room temperature (25°C or lower).

1.1.3 rhEGF eye drops (trade name: Yibei)

Manufacturer: Guilin Pavay Gene Pharmaceuticals Co. Ltd.
Specification: 20000 IU (40 μl)/2 ml/bottle
Storage condition: stored at 4°C to 25°C.

1.1.4 Artificial tears prepared according to Example 7

Specification: 0.8 mL/bottle
Recombinant human lysozyme: 1.2 mg: 120000 U
rhEGF: 16 μg: 8000IU;
Sodium hyaluronate: 0.8 mg;
Appearance: colorless transparent clear liquid
Storage condition: stored at room temperature (25°C or lower).

1.2 Laboratory animals

SD rats, each weighing 193 g to 234 g, including both male and female, were purchased from the Laboratory animal center of the Fourth Military Medical University.

2. Test design and method

2.1 Establishment and evaluation of dry-eye animal model accompanied by severe corneal damage

2.1.1 Model establishment

A rat model of chemical bacteriostatic-induced dry-eye and corneal damage was established by dropwise addition of 0.1% benzalkonium chloride to both eyes, three times a day, with one drop (about 35 µl) each time, for 10 successive days.

2.1.2 Evaluation of the model

24 SD rats were subjected to a tear secretion test and a slit lamp examination of fluorescein sodium staining before the establishing a model and 10 days after the establishing a model.

2.1.2.1 Tear secretion test

A surface anesthetic was added dropwise onto the ocular surface of a rat. After one minute counted, a piece of tear test paper (a 5 mm × 35 mm piece of test paper for tear assays was cut into 2.0 mm×35 mm with a paper knife) was bent by 5 mm at one end, which was inserted 1/3 deep into the of the rat's lower eyelid, with the rest being pendent naturally. After 5 minutes counted, the length of the portion of the filter paper that was wetted with tears was measured. A length longer than 10 mm/5 min indicates normal secretion, while a length below 5 mm/5 min indicates a dry eye.

2.1.2.2 Corneal epithelial fluorescein sodium staining

A 10 µl solution of 1% fluorescein sodium was taken with a pipette and dropped into the conjunctival sac of a rat, and the eyes of the rat were manually made to wink to uniformly distribute the fluorescein on the ocular surface of the rat eyes. The eye lids were fixed and photographed under a cobalt blue light lamp, and the staining of the corneal epithelium was observed. The scoring method was a 12-point method with reference to the expert consensus standards for clinical diagnosis and treatment of ophthalmo xerosis (2013): the cornea was divided into 4 quadrants, with each quadrant given a score of 0-3 points, in which a quadrant having no staining was given 0 point, a quadrant having 1-30 stained spots was given 1 point, a quadrant having more than 30 stained spots without merging of staining was given 2 points, and a quadrant showing merging of stained spots, filament-like bodies and ulcers in the cornea was given 3 points.

2.2 Evaluation of efficacy of artificial tears

2.2.1 Grouping

After successful establishing a model, the SD rats were grouped into a sodium hyaluronate control group; a recombinant human lysozyme control group; an rhEGF control group; and an artificial tear group, with 6 rats per group, 24 rats in total.

2.2.2 Treatment regime

In all groups, the left eyes of the SD rats were allowed to recover naturally, and the sodium hyaluronate control, the recombinant human lysozyme control, the rhEGF control, or the artificial tears were added dropwise to the right eyes in corresponding groups.

    Route: Instillation inside the orbits
    Frequency: 3 times per day.
    Volume: one drop (about 35 µl) / eye.
    Period: continuous administration for 5 days.

2.2.3 Examination indicators

After 5 days of continuous administration, the tear secretion test and the slit-lamp examination of fluorescein sodium staining were conducted (by the same methods as described above). At the end of the experiment, the rats were sacrificed by cervical dislocation, and the eyeballs were extirpated, perforated on the retina, and fixed in a fixing solution for 2 hours. Afterwards, the posterior part of the eyeball behind the corneal sclera edge was removed, followed by further fixing for 24 hours, conventional dehydration, paraffin embedding, sectioning, and HE staining. The histological observation was performed under a microscope.

2.3 Statistical analysis

The average value and standard deviation of the measurement data were calculated for each group, and then analyzed by ANOVA.

3. Results

3.1 Evaluation of chemical bacteriostatic-induced dry-eye and corneal damage rat model

3.1.1 Tear secretion test

As compared between before and after the establishing a model of rats, the tear-wetted length of the filter paper showed highly significant difference ($p < 0.01$) (see Table 10).

3.1.2 Corneal epithelial fluorescein sodium staining

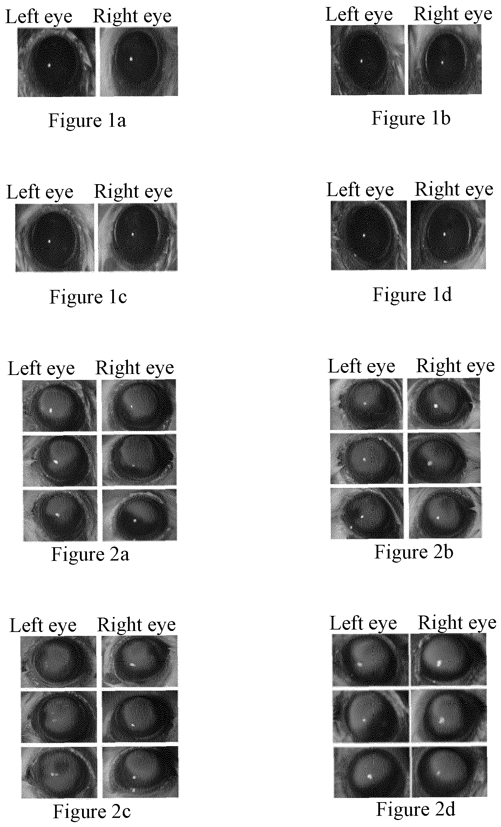

The photographs of fluorescein sodium staining before establishing a model are shown in Figures 1a to 1d, showing that the left and right eyes of all rats were not apparently stained, and the corneas were all normal. 10 days after establishing a model, the photographs of fluorescein sodium staining are shown in Figures 2a to 2d, showing that the corneas of the left and right eyes of all rats were apparently damaged. According to the scoring standard, the scores of both eyes of the rats before establishing a model were significantly different from those after establishing a model ($p<0.01$) (see in Table 11).

3.1.3 Summary

Through the tear secretion test and the fluorescein staining examination, there was a highly significant difference in eyes between the rats before and after the establishing a model, all with statistical significance, indicating that the establishing a model was successful.

3.2 Evaluation of efficacy of artificial tears

3.2.1 Tear secretion test

After 5 days of administration, the tear-wetted length of the filter paper in the right eyes in both the sodium hyaluronate control group and the artificial tear group was significantly increased and showed significant difference as compared to the naturally recovered left eyes in the same group ($p<0.05$). In the recombinant human lysozyme control group and the rhEGF control group, there was no significant difference in the tear-wetted length of the filter paper between the left eyes and the right eyes. The tear-wetted length of the filter paper in the treated right eyes in the artificial tear group was significantly increased and showed significant difference as compared to the other three groups ($p<0.05$) (see Table 12).

3.2.2 Corneal epithelial fluorescein sodium staining

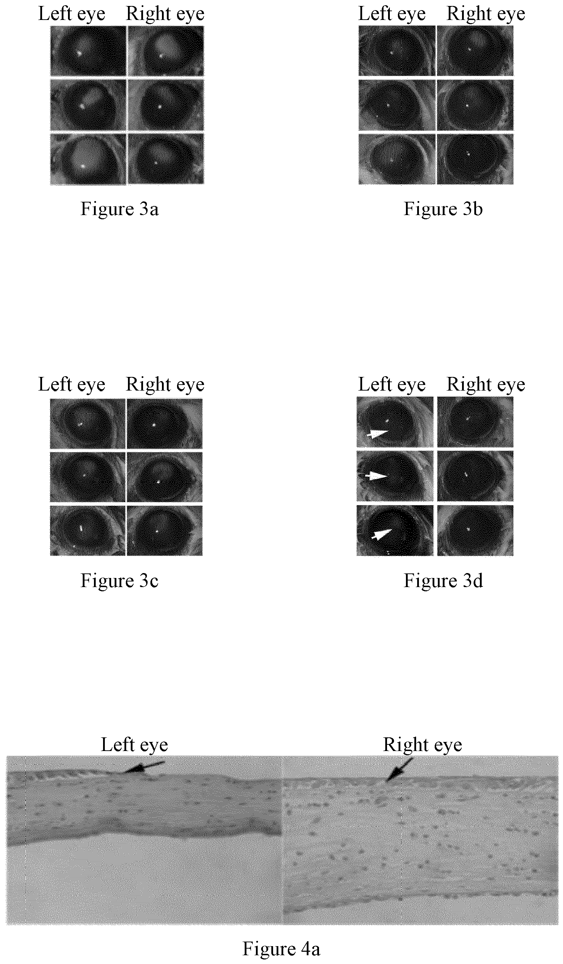

As shown in Figures 3a to 3d, after 5 days of administration, the right eyes of the rats in the sodium hyaluronate control group, the recombinant human lysozyme control group, and the rhEGF control group still showed a certain area of pieces stained with fluorescein sodium; the cornea of the right eyes of the rats in the artificial tear group was substantially not stained, had a few spot-like injuries under slip lamp examination, but still showed white turbidity and poor transparency (as compared to the photographs before the establishing a model). According to the scoring standard, after administration, the scores of the right eyes in the sodium hyaluronate control group, the rhEGF control group, and the artificial tear group were significantly different as compared to those of the naturally recovered left eyes in the same group ($p<0.05$). The scores of the right eyes in the artificial tear group was significantly different from the scores of the right eyes in the other three groups ($p<0.05$) (see Table 13).

3.2.3 Histological examination

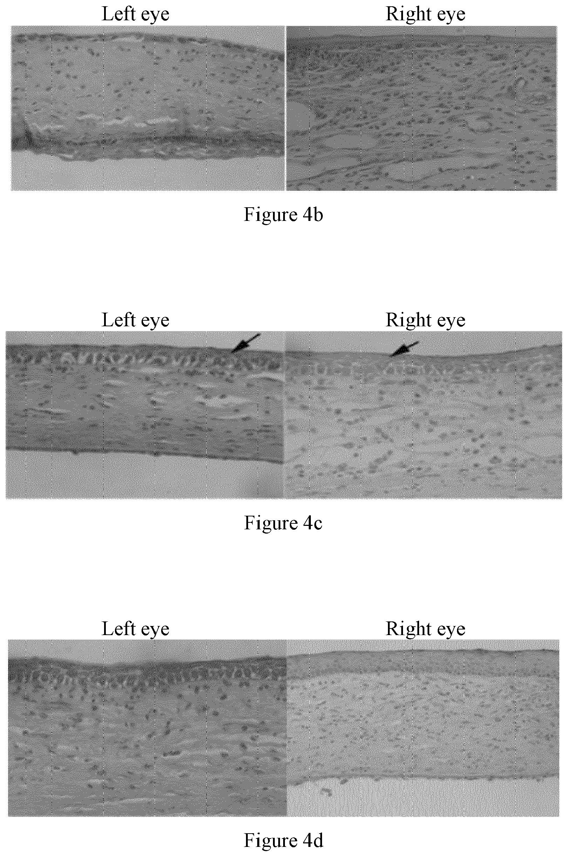

After 5 days of treatment, the histopathological sections of the naturally recovered left eyes in each group showed that the epithelial cell layer at the corneal areas stained with fluorescein sodium (mostly located in the center of the cornea) was thinned or completely absent, and the shrunk, regular elastic fiber structure in the matrix layer was disordered, and even infiltrated by inflammatory cells. As shown in Figures 4a to 4d, in the treated right eyes in the sodium hyaluronate control group, the recombinant human lysozyme reference group, and the rhEGF control group, the epithelial cell layer at the corneal areas stained with fluorescein sodium showed certain recovery, but was still thin; while in the right eyes of the rats in the artificial tear group, although the corneal epithelial cell layer still showed a trend of turning disordered, the number of cells and the thickness of corneal epithelium recovered well. However, in the cornea of right eyes of the rats in the artificial tear group, phenomena of disordered arrangement and an irregular structure can still been observed in the matrix layer fibers.

4. Conclusion

The above results demonstrate that:

(1) benzalkonium chloride (at a concentration of 0.1%), a chemical bacteriostatic commonly used in eye drops, caused dry eyes and damage to corneal epithelial cells when applied to eyes for a long period; and
(2) as compared to the sodium hyaluronate control group, the recombinant human lysozyme control group, and the rhEGF control group, 5 days of treatment with the artificial tears produced significant efficacy and better effects of improving tear secretion amount and promoting repair of corneal epithelia cells on the rat model of dry eye and severe corneal damage induced with 0.1% benzalkonium chloride, although the corneal damage induced with benzalkonium chloride was not completely cured.

Table 10. Length (mm) of the portion of filter paper wetted with tears before and after the establishing a model

| Group | n | Before Establishing a model | 10 Days After Establishing a model |
|---|---|---|---|
| Left eyes | 24 | 12.86±2.65 | 3.55±1.37** |
| Right eyes | 24 | 12.41±3.08 | 3.38±1.63** |
| Note: Before establishing a model v.s. after establishing a model, ** P<0.01 | | | |

Table 11. Scores of fluorescein sodium staining of corneal epithelium before and after the establishing a model

| Group | n | Before Surgery | 1 Week After Surgery |
|---|---|---|---|
| Left eyes | 24 | 1.11±0.33 | 12.00±0.00** |
| Right eyes | 24 | 1.17±0.38 | 12.00±0.00** |
| Note: Before establishing a model v.s. after establishing a model, ** P<0.01 | | | |

Table 12. Tear secretion test results of 5-day treatment (mm)

| Group | Sodium hyaluronate control group | Recombinant human lysozyme control group | rhEGF control group | Artificial tear group |
|---|---|---|---|---|
| Left eyes (naturally recovered) | 8.53±1.92 | 9.22±2.88 | 8.12±2.76 | 9.35±2.67 |
| Right eyes (treated) | 11.54±2.78*+ | 10.36±2.72+ | 9.34±1.82 | 13.25±1.67*#△+ |
| Note: n=6. As compared to the left eyes in the same group: *p<0.05; as compared to the right eyes in the sodium hyaluronate control group: #p<0.05; as compared to the right eyes in the recombinant human lysozyme control group: △p<0.05; as compared to the right eyes in the rhEGF control group: +p<0.05. | | | | |

Table 13. Scores of fluorescein sodium staining of corneal epithelium of 5-day treatment

| Group | Sodium hyaluronate control group | Recombinant human lysozyme control group | rhEGF control group | Artificial tear group |
|---|---|---|---|---|
| Left eyes (naturally recovered) | $8.33\pm3.87$ | $6.83\pm2.66$ | $6.94\pm3.82$ | $4.32\pm2.78$ |
| Right eyes (treated) | $6.86\pm3.78$* | $5.88\pm2.51$ | $4.54\pm2.12$*# | $2.15\pm1.87$*#△+ |

Note: n=6. As compared to the left eyes in the same group: *$p<0.05$; as compared to the right eyes in the sodium hyaluronate control group: #$p<0.05$; as compared to the right eyes in the recombinant human lysozyme control group: △$p<0.05$; as compared to the right eyes in the rhEGF control group: +$p<0.05$.

Example 17: Test of pharmacodynamic effects of artificial tears on human dry eyes

1. Test materials

Control:

rhEGF eye drops (trade name: Yibei)
Manufacturer: Guilin Pavay Gene Pharmaceuticals Co. Ltd.
Specification: 20000 IU (40 $\mu$l)/2 ml/bottle
Storage condition: stored at 4°C to 25°C.

Test Sample:

Artificial tears prepared according to Example 7
Specification: 0.8 ml
Recombinant human lysozyme: 1.2 mg: 120000 U
rhEGF: 16 $\mu$g: 8000IU;
Sodium hyaluronate: 0.8 mg;
Appearance: colorless transparent clear liquid
Storage condition: stored at room temperature (25°C or lower).

2. Test design and method

2.1 Subjects

44 subjects were selected, including 14 males and 30 females, aged 37-50 years. The selection criteria were as follows: (1) employees at positions involving long-term working in front of a computer screen with excessive use of eyes, such as financial staff, legal staff, office administrative personnel, lamp inspectors responsible for quality check, and the like; (2) persons having subjective uncomfortable symptoms in eyes, such as obvious itching in eyes, eye dryness, photophobia, eye fatigue, foreign body sensation, burning sensation, soreness, episodic pain in eyes, eye redness, conjunctival congestion in eye lid and eyeball, and the like; (3) subjects to be excluded: persons having systemic diseases, or having taken glucocorticoids, non-steroidal anti-inflammatory drugs, and immunosuppressive agents.

2.2 Test methods

The 44 subjects were treated with the rhEGF eye drops for 15 days, 4 administrations per day, with one drop per administration in the left eye; and treated with the artificial tears for 15 days, 4 administrations per day, with one drop per administration in the right eye. On the 5th, 10th, and 15th days since administration, the subjects were observed and the subjective symptoms were recorded.

2.3 Data statistics

The 44 subjects were asked to fill a questionnaire about dry eye symptoms before and after the treatment. The questionnaire included 10 items to be scored, i.e. itching, eye dryness, photophobia, eye fatigue, foreign body sensation, burning sensation, soreness, episodic pain in eyes, eye redness, and conjunctival congestion in eye lid and eyeball, and each item was given a score of 0 to 4 from mild to severe in terms of severity and duration in each of the left and right eyes. All data were statistically analyzed using the SPSS20 software. The data were expressed as mean $\pm$ standard deviation, and compared by t-test. $p < 0.05$ was considered statistically significant.

3. Test results

Under the conditions of this test, there were no significant differences in the score of subjective symptoms between the left and right eyes of the 44 subjects before treatment. After 5, 10, and 15 days of treatment, the subjects subjectively reported a marked difference between the left and right eyes, and the data statistics showed a marked difference between the left and right eyes (the results are shown in Table 14) with statistical significance.
As compared with the rhEGF eye drop control, the tested artificial tears can markedly and more effectively improve the related symptoms of tear secretion-deficient ophthalmo xerosis caused by excessive use of eyes and reduced nictations in people's working and daily life, and promote repair of damaged corneal cells.

Table 14. Summary of scores in the questionnaire about subjective dry eye symptoms

|  | Before treatment | 5-day treatment | 10-day treatment | 15-day treatment |
|---|---|---|---|---|
| Left eyes | 23.6±2.83 | 21.7±3.66 | 18.6±4.03 | 15.5±3.73 |
| Right eyes | 24.1±2.04 | 16.7±3.92* | 10. 1±3.3 3 * | 8.66±2.25* |
| Note: n=44; Right eye compared to left eye, *p<0.05 | | | | |

**Claims**

1. Artificial tears containing a recombinant human lysozyme and a recombinant human epidermal growth factor (rhEGF), said artificial tears comprising main components and an auxiliary material, wherein the main components are a recombinant human lysozyme, an rhEGF, and sodium hyaluronate, and the contents of the recombinant human lysozyme, the rhEGF, and the sodium hyaluronate are 0.075%-0.300%, 0.002%-0.004%, and 0.10%-0.30%, respectively, based on the total mass of the novel artificial tears;

   wherein the auxiliary material comprises a pH stabilizer, and the pH stabilizer is a buffer consisting of disodium hydrogen phosphate and sodium dihydrogen phosphate;
   the auxiliary material comprises sodium chloride, and based on the total mass of the artificial tears, the content of sodium chloride is 0.70%-0.76% as measured in terms of pure solid sodium chloride; wherein the novel artificial tears have a pH of 6.4 to 6.6 and an osmolality of 285 to 310 mOsmol/kg.

2. The artificial tears according to claim 1, wherein the recombinant human lysozyme has an amino acid sequence completely identical to that of natural human lysozyme; and the rhEGF has an amino acid sequence completely identical to that of natural human EGF.

3. The artificial tears according to claim 1, wherein the recombinant human lysozyme is contained in an amount of 0.150% to 0.300% based on the total mass of the artificial tears.

4. The artificial tears according to claim 1, wherein the novel artificial tears have a pH of 6.5.

5. The artificial tears according to claim 1, wherein the disodium hydrogen phosphate is disodium hydrogen phosphate dodecahydrate, and the sodium dihydrogen phosphate is sodium dihydrogen phosphate dihydrate.

6. The artificial tears according to claim 5, wherein the mass ratio of disodium hydrogen phosphate dodecahydrate and sodium dihydrogen phosphate dihydrate is 20:9.

7. The artificial tears according to any one of claim 1, wherein based on the total amount of the novel artificial tears of 10,000-20,000 parts by weight, the novel artificial tears comprise: 7.5-60.0 parts of the recombinant human lysozyme, 0.2 to 0.8 parts of the rhEGF, 10.0-60.0 parts of the sodium hyaluronate, 20.0-40.0 parts of the disodium hydrogen phosphate dodecahydrate, 9.0-18.0 parts of the sodium dihydrogen phosphate dihydrate, 70.0-152.0 parts of the sodium chloride, and 9,840.6-19,754.6 parts of water for injection;

preferably, wherein based on the total amount of the novel artificial tears of 10,000 parts by weight, the novel artificial tears comprise: 15.0-30.0 parts of the recombinant human lysozyme, 0.2 to 0.4 parts of the rhEGF, 10.0-30.0 parts of the sodium hyaluronate, 20.0 parts of the disodium hydrogen phosphate dodecahydrate, 9.0 parts of the sodium dihydrogen phosphate dihydrate, 70.0-75.0 parts of the sodium chloride, and 9,840.6-9,870.8 parts of the water for injection.

8. The artificial tears according to claim 7, wherein based on the total amount of the novel artificial tears of 10,000 parts by weight, the novel artificial tears comprising: 7.5 parts of the recombinant human lysozyme, 0.2 parts of the rhEGF, 10.0 parts of sodium hyaluronate, 20.0 parts of the disodium hydrogen phosphate dodecahydrate, 9.0 parts of the sodium dihydrogen phosphate dihydrate, 76.0 parts of the sodium chloride, and 9,877.3 parts of the water for injection; or

based on the total amount of the novel artificial tears of 10,000 parts by weight, the novel artificial tears comprising: 15.0 parts of the recombinant human lysozyme, 0.2 parts of the rhEGF, 10.0 parts of the sodium hyaluronate, 20.0 parts of the disodium hydrogen phosphate dodecahydrate, 9.0 parts of the sodium dihydrogen phosphate dihydrate, 75.0 parts of the sodium chloride, and 9,870.8 parts of the water for injection; or
based on the total amount of the novel artificial tears of 10,000 parts by weight, the novel artificial tears comprise: 15.0 parts of the recombinant human lysozyme, 0.2 parts of the rhEGF, 30.0 parts of the sodium hyaluronate, 20.0 parts of the disodium hydrogen phosphate dodecahydrate, 9.0 parts of the sodium dihydrogen phosphate dihydrate, 72.0 parts of the sodium chloride, and 9,853.8 parts of the water for injection; or
based on the total amount of the novel artificial tears of 20,000 parts by weight, the novel artificial tears comprise: 60.0 parts of the recombinant human lysozyme, 0.4 parts of the rhEGF, 20.0 parts of the sodium hyaluronate, 40.0 parts of the disodium hydrogen phosphate dodecahydrate, 18.0 parts of the sodium dihydrogen phosphate dihydrate, 148.0 parts of the sodium chloride, and 19,713.6 parts of the water for injection; or
based on the total amount of the novel artificial tears of 20,000 parts by weight, the novel artificial tears comprise: 60.0 parts of the recombinant human lysozyme, 0.8 parts of the rhEGF, 60.0 parts of the sodium hyaluronate, 40.0 parts of the disodium hydrogen phosphate dodecahydrate, 18.0 parts of the sodium dihydrogen phosphate dihydrate, 140.0 parts of the sodium chloride, and 19,681.2 parts of the water for injection.

**Patentansprüche**

1. Künstliche Tränen, die ein rekombinantes humanes Lysozym und einen rekombinanten humanen epidermalen Wachstumsfaktor (rhEGF) enthalten, wobei die künstlichen Tränen Hauptbestandteile und ein Hilfsmaterial umfassen, wobei die Hauptbestandteile ein rekombinantes humanes Lysozym, ein rhEGF und Natriumhyaluronat sind und die Gehalte des rekombinanten humanen Lysozyms, des rhEGFs und des Natriumhyaluronats 0,075 % bis 0,300 %, 0,002 % bis 0,004 % bzw. 0,10 % bis 0,30 %, bezogen auf die Gesamtmasse der neuen künstlichen Tränen, betragen;

wobei das Hilfsmaterial einen pH-Stabilisator umfasst, der aus Dinatriumhydrogenphosphat und Natriumdihydrogenphosphat besteht,
das Hilfsmaterial Natriumchlorid umfasst und, bezogen auf die Gesamtmasse der künstlichen Tränen, der Gehalt an Natriumchlorid 0,70 %-0,76 %, gemessen als reines festes Natriumchlorid, beträgt;
wobei die neuen künstlichen Tränen einen pH-Wert von 6,4 bis 6,6 und eine Osmolalität von 285 bis 310 mOsmol/kg aufweisen.

2. Künstliches Tränen nach Anspruch 1, wobei das rekombinante humane Lysozym eine Aminosäuresequenz aufweist, die vollständig mit derjenigen des natürlichen humanen Lysozyms identisch ist und das rhEGF eine Aminosäuresequenz aufweist, die vollständig mit derjenigen des natürlichen humanen EGFs identisch ist.

3. Künstliche Tränen nach Anspruch 1, wobei das rekombinante humane Lysozym in einer Menge von 0,150 % bis 0,300 %, bezogen auf die Gesamtmasse der künstlichen Tränen, enthalten ist.

4. Künstliche Tränen nach Anspruch 1, wobei die neuen künstlichen Tränen einen pH-Wert von 6,5 aufweisen.

5. Künstliche Tränen nach Anspruch 4, wobei das Dinatriumhydrogenphosphat Dinatriumhydrogenphosphat-Dodecahydrat ist und das Nnatriumdihydrogenphosphat Natriumdihydrogenphosphat-Dihydrat ist.

6. Künstliche Tränen nach Anspruch 4, wobei das Gewichtsverhältnis von Dinatriumhydrogenphosphat-Dodecahydrat zu Natriumdihydrogenphosphat-Dihydrat 20:9 beträgt.

7. Künstliche Tränen nach Anspruch 1, wobei, bezogen auf die Gesamtmenge der neuartigen künstlichen Tränen von 10.000-20.000 Gewichtsteilen, die neuartigen künstlichen Tränen 7,5-60,0 Teile des rekombinanten menschlichen Lysozyms, 0,2 bis 0,8 Teile des rhEGFs, 10,0-60,0 Teile des Natriumhyaluronats, 20,0-40,0 Teile des Dinatriumhydrogenphosphat-Dodecahydrats, 9,0-18,0 Teile des Natriumdihydrogenphosphat-Dihydrats, 70,0-152,0 Teile des Natriumchlorids und 9.840,6-19.754,6 Teile Wasser für Injektionszwecke umfassen;
vorzugsweise, wobei, bezogen auf die Gesamtmenge der neuen künstlichen Tränen von 10.000 Gewichtsteilen, die neuen künstlichen Tränen 15,0-30,0 Teile des rekombinanten menschlichen Lysozyms, 0,2 bis 0,4 Teile des rhEGFs, 10,0-30,0 Teile des Natriumhyaluronats, 20,0 Teile des Dinatriumhydrogenphosphat-Dodecahydrats, 9,0 Teile des Natriumdihydrogenphosphat-Dihydrats, 70,0-75,0 Teile des Natriumchlorids und 9.840,6-9.870,8 Teile des Wassers für Injektionszwecke umfassen.

8. Künstliche Tränen nach Anspruch 7, wobei, bezogen auf die Gesamtmenge der neuartigen künstlichen Tränen von 10.000 Gewichtsteilen, die neuartigen künstlichen Tränen 7,5 Teile des rekombinanten humanen Lysozyms, 0,22 Teile des rhEGFs, 10,0 Teile des Natriumhyaluronats, 20,0 Teile des Dinatriumhydrogenphosphatdodecahydrats, 9,0 Teile des Natriumdihydrogenphosphatdihydrats, 76,0 Teile des Natriumchlorids und 9.877,3 Teile des Wassers für Injektionszwecke umfassen, oder,

bezogen auf die Gesamtmenge der neuartigen künstlichen Tränen von 10.000 Gewichtsteilen, die neuartigen künstlichen Tränen 15,0 Teile des rekombinanten humanen Lysozyms, 0,2 Teile des rhEGFs, 10,0 Teile des Natriumhyaluronats, 20,0 Teile des Dinatriumhydrogenphosphatdodecahydrats, 9,0 Teile des Natriumdihydrogenphosphatdihydrats, 75,0 Teile Natriumchlorids und 9.870,8 Teile des Wassers für Injektionszwecke umfassen, oder,
bezogen auf die Gesamtmenge der neuartigen künstlichen Tränen von 10.000 Gewichtsteilen, die neuartigen künstlichen Tränen 15,0 Teile des rekombinanten humanen Lysozyms, 0,2 Teile des rhEGFs, 30,0 Teile des Natriumhyaluronats, 20,0 Teile des Dinatriumhydrogenphosphat-Dodecahydrats, 9,0 Teile des Natriumdihydrogenphosphat-Dihydrats, 72,0 Teile des Natriumchlorids und 9.853,8,0 Teile des Wassers für Injektionszwecke umfassen, oder,
bezogen auf die Gesamtmenge der neuartigen künstlichen Tränen von 20.000 Gewichtsteilen, die neuartigen künstlichen Tränen 60,0 Teile des rekombinanten humanen Lysozymes, 0,4 Teile des rhEGFs, 20,0 Teile des Natriumhyaluronats, 40,0 Teile des Dinatriumhydrogenphosphat-Dodecahydrats, 18,0 Teile des Natriumdihydrogenphosphat-Dihydrats, 148,0 Teile des Natriumchlorids und 19.713,6 Teile des Wassers für Injektionszwecke umfassen, oder,
bezogen auf die Gesamtmenge der neuartigen künstlichen Tränen von 20.000 Gewichtsteilen, die neuartigen künstlichen Tränen 60,0 Teile des rekombinanten humanen Lysozyms, 0,8 Teil des rhEGFs, 60,0 Teile des Natriumhyaluronats, 40,0 Teile des Dinatriumhydrogenphosphat-Dodecahydrats, 18,0 Teile des Natriumdihydrogenphosphat-Dihydrats, 140,0 Teile des Natriumchlorids und 19.681,2 Teile des Wassers für Injektionszwecke umfassen.

## Revendications

1. larmes artificielles contenant un lysozyme humain recombinant, lesdites larmes artificielles comprenant des composants principaux et un matériau auxiliaire, dans lesquelles les composants principaux sont un lysozyme humain recombinant, et facteur de croissance épidermique humain recombinant (rhEGF) et un hyaluronate de sodium, et les teneurs en lysozyme humain recombinant, en rhEGF et en hyaluronate de sodium sont respectivement de 0,075 % à 0,300 %, de 0,002 % à 0,004 % et de 0,10 % à 0,30 %, sur la base de la masse totale des nouvelles larmes artificielles ;

dans laquelle la matière auxiliaire comprend un stabilisateur de pH constitué d'hydrogénophosphate disodique et de dihydrogénophosphate de sodium ;
la matière auxiliaire comprend du chlorure de sodium, et sur la base de la masse totale des larmes artificielles, la teneur en chlorure de sodium est de 0,70 % à 0,76 %, mesurée en termes de chlorure de sodium solide pur ;

dans lesquelles les nouvelles larmes artificielles ont un pH de 6,4 à 6,6 et une osmolalité de 285 à 310 mOsmol/kg.

2. Larmes artificielles selon la revendication 1, dans lesquelles le lysozyme humain recombinant a une séquence d'acides aminés complètement identique à celle du lysozyme humain naturel et dans lesquelle lesquelles le rhEGF a une séquence d'acides aminés complètement identique à celle du EGF humain naturel.

3. Larmes artificielles selon la revendication 1, dans lesquelles le lysozyme humain recombinant est contenu dans une quantité de 0,150 % à 0,300 % par rapport à la masse totale des larmes artificielles.

4. Larmes artificielles selon la revendication 1, dans lesquelles les nouvelles larmes artificielles ont un pH de 6,5.

5. Larmes artificielles selon la revendication 4, dans lesquelles l'hydrogénophosphate disodique est de l'hydrogénophosphate disodique dodécahydraté, et le dihydrogénophosphate de sodium est du dihydrogénophosphate de sodium dihydraté.

6. Larmes artificielles selon la revendication 5, dans lesquelles le rapport de masse entre l'hydrogénophosphate de sodium dodécahydraté et le dihydrogénophosphate de sodium dihydraté est de 20:9.

7. Les larmes artificielles selon la revendication 1, dans lesquelles, sur la base de la quantité totale des nouvelles larmes artificielles de 10 000-20 000 parties en poids, les nouvelles larmes artificielles comprennent : 7,5-60,0 parties du lysozyme humain recombinant, 0,2-0,8 parties du rhEGF, 10,0-60,0 parties de hyaluronate de sodium, 20,0-40,0 parties d'hydrogénophosphate dodécahydraté d'isodium, 9,0-18,0 parties de dihydrogénophosphate de sodium dihydraté, 70,0-152,0 parties de chlorure de sodium, et 9,840,6-19,754,6 parties d'eau pour injection ;
de préférence, dans laquelle, sur la base de la quantité totale des nouvelles larmes artificielles de 10 000 parties en poids, les nouvelles larmes artificielles comprennent : 15,0-30,0 parties du lysozyme humain recombinant, 0,2-0,4 parties du rhEGF, 10,0-30,0 parties du hyaluronate de sodium, 20,0 parties de l'hydrogénophosphate disodique dodécahydraté, 9,0 parties du dihydrogénophosphate de sodium dihydraté, 70,0-75,0 parties du chlorure de sodium, et 9,840,6-9,870,8 parties de l'eau pour injection.

8. Les larmes artificielles selon la revendication 7, dans lesquelles, sur la base de la quantité totale des nouvelles larmes artificielles de 10 000 parties en poids, les nouvelles larmes artificielles comprennent : 7,5 parties de lysozyme humain recombinant, 0,2 parties du rhEGF, 10,0 parties de hyaluronate de sodium, 20,0 parties d'hydrogénophosphate disodique dodécahydraté, 9,0 parties de dihydrogénophosphate de sodium dihydraté, 76,0 parties de chlorure de sodium, et 9 877,3 parties d'eau pour injection ; ou

sur la base de la quantité totale des nouvelles larmes artificielles de 10 000 parties en poids, les nouvelles larmes artificielles comprenant : 15,0 parties de lysozyme humain recombinant, 0,2 parties du rhEGF, 10,0 parties de hyaluronate de sodium, 20,0 parties d'hydrogénophosphate disodique dodécahydraté, 9,0 parties de dihydrogénophosphate de sodium dihydraté, 75,0 parties de chlorure de sodium, et 9 870,8 parties d'eau pour injection ; ou sur la base d'une quantité totale de larmes artificielles nouvelles de 10 000 parties en poids, les larmes artificielles nouvelles comprennent : 15,0 parties de lysozyme humain recombinant, 0,2 parrties du rhEGF, 30,0 parties de hyaluronate de sodium, 20,0 parties d'hydrogénophosphate disodique dodécahydraté, 9,0 parties de dihydrogénophosphate de sodium dihydraté, 72,0 parties de chlorure de sodium, et 9 853,8 parties d'eau pour injection ; ou sur la base d'une quantité totale de nouvelles larmes artificielles de 20 000 parties en poids, les nouvelles larmes artificielles comprennent : 60,0 parties de lysozyme humain recombinant, 0,4 parties du rhEGF, 20,0 parties de hyaluronate de sodium, 40,0 parties d'hydrogénophosphate disodique dodécahydraté, 18,0 parties de dihydrogénophosphate de sodium dihydraté, 148,0 parties de chlorure de sodium, et 19 713,6 parties d'eau pour injection ; ou
sur la base d'une quantité totale de larmes artificielles nouvelles de 20 000 parties en poids, les larmes artificielles nouvelles comprennent : 60,0 parties de lysozyme humain recombinant, 0,8 parties du rhEGF, 60,0 parties de hyaluronate de sodium, 40,0 parties d'hydrogénophosphate disodique dodécahydraté, 18,0 parties de dihydrogénophosphate de sodium dihydraté, 140,0 parties de chlorure de sodium et 19 681,2 parties d'eau pour préparations injectables.

Left eye    Right eye

Figure 1a

Left eye    Right eye

Figure 1b

Left eye    Right eye

Figure 1c

Left eye    Right eye

Figure 1d

Left eye    Right eye

Figure 2a

Left eye    Right eye

Figure 2b

Left eye    Right eye

Figure 2c

Left eye    Right eye

Figure 2d

Left eye   Right eye

Figure 3a

Left eye   Right eye

Figure 3b

Left eye   Right eye

Figure 3c

Left eye   Right eye

Figure 3d

Left eye                Right eye

Figure 4a

Left eye              Right eye

Figure 4b

Left eye              Right eye

Figure 4c

Left eye              Right eye

Figure 4d

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070280924 A1
- US 7655698 B2
- US 20080213188 A1

- US 20170246218 A1
- CN 102188695 A
- CN 102552889 A

**Non-patent literature cited in the description**

- **SAAEHA RAUZ et al.** *Serum eye drops, amniotic membrane and limbal epithelial stem cells-tools in the treatment of ocular surface disease,* 22 April 2009

- Pharmacopoeia of People's Republic of China. 2015